# EUROPEAN PATENT APPLICATION

(11) **EP 1 182 210 A1**
(43) Date of publication of application: **27.02.2002**
(21) Application number: 00117735.1
(22) Date of filing: 17.08.2000
(51) Int. Cl.: C07K 14/47, C07K 16/18, C07K 9/00, C12N 5/06, G01N 33/68, G01N 33/574

(54) **Epitopes of tumor-associated MUC1 antigen**

(71) Applicant: BASTERT, Gunter, Prof.Dr.med.Dr.h.c., D-69115 Heidelberg (DE)
(72) Inventor: Bastert, Gunther, Prof. Dr.med Dr.h.c., 69115 Heidelberg (DE); Kaul, Sepp, Dr., 69115 Heidelberg (DE)
(74) Representative: Pätzold, Herbert, Dr.-Ing.

(57) **Abstract**

The invention devises the structures and the uses of the carbohydrate-containing epitopes of tumor-associated underglycosylated MUC1 antigens that are specifically bound by Mabs 7F11 and 1E4 that have been disclosed in the published International Patent Application WO 99/40881.

SEQUENZPROTOKOLL

<110>	BASTERT, Gunther

<120>	Epitopes of Tumor-Associated MUC1 Antigen

<130>	3294

<160>	6

<210>	1
<211>	8
<212>	PRT
<213>	human

>220>
<221>	CARBOHYD
<222>	3
<223>	Position 2: aspartate or glutamate; Position 3: threonine or serine, with single
	alpha-GalNAc residue in O-glycosidic linkage; position 4: arginine, lysine or
	histidine; position 6: glycine, serine, tyrosine, threonine, cysteine, asparagine
	or glutamine; position 8: serine, glycine, tyrosine, threonine, cysteine,
	asparagine, glutamine or absent

<400>	1

Pro Xaa Xaa Xaa Pro Xaa Pro Xaa
                5

<210>	2
<211>	8

<212>	PRT
<213>	human

>220>
<221>	CARBOHYD
<222>	3
<223>	CARBOHYD/ single alpha-GalNAc residue in O-glycosidic linkage

<400>	2

Pro Asp Thr Arg Pro Ala Pro Gly
                5

<210>	3
<211>	10
<212>	PRT
<213>	human

>220>
<221>	CARBOHYD
<222>	3
<223>	CARBOHYD/ single alpha-GalNAc residue in O-glycosidic linkage

<400>	3

Pro Asp Thr Arg Pro Ala Pro Gly Ser Thr
                5                   10

<210>	4
<211>	11
<212>	PRT
<213>	human

>220>
<221>	CARBOHYD
<222>	4
<223>	CARBOHYD/ single alpha-GalNAc residue in O-glycosidic linkage

<400>	4

Ala Pro Asp Thr Arg Pro Ala Pro Gly Ser Thr
                5                   10

<210>	5
<211>	12
<212>	PRT
<213>	human

>220>
<221>	CARBOHYD
<222>	5
<223>	CARBOHYD/ single alpha-GalNAc residue in O-glycosidic linkage

<400>	5

Ser Ala Pro Asp Thr Arg Pro Ala Pro Gly Ser Thr
                5                   10

<210>	6
<211>	20
<212>	PRT
<213>	human

>220>

<221>	CARBOHYD
<222>	3
<223>	CARBOHYD/ single alpha-GalNAc residue in O-glycosidic linkage

<400>	3

Pro Asp Thr Arg Pro Ala Pro Gly Ser Thr
                5                   10

Ala Pro Pro Ala His Gly Val Thr Ser Ala
                15                  20

## Description

The present invention relates to epitopes of tumor-associated mucin MUC1 antigen, their structures and their use to raise specific in vivo-immune responses, to generate specifically cross-reacting antisera, to select specifically cross-reacting monoclonal antibodies, recombinant antibodies, antibody-like proteins containing antibodies or active parts of antibodies, e.g. complementarity-determining regions, and chimeric proteins containing antibodies or active parts of antibodies and in addition heterologous proteins or parts thereof, which can be obtained by molecular biological methods, and to generate tumor specific effector T cells, NK cells and B cells. The present invention relates in addition to the use of said antisera, monoclonal antibodies, recombinant antibodies, antibody-like proteins, chimeric proteins, tumor specific effector T cells, NK cells and B cells for diagnostic and therapeutic purposes.

Mucins are present at the secretions of epithelial surfaces of the respiratory, gastrointestinal and urogenital tract. In sebaceous and salivary glands, mucins form the major components of the mucinous secretions of these tissues. Mucins are secreted in large amounts by differentiated breast epithelium in milk, and also in other organs with secretorial epithelia. The physiological function of mucins has not yet been fully elucidated. It is very probable that in many organs they protect epithelial cells against changes in pH, against mechanical and microbial damage.

MUC1 is a transmembrane glycoprotein, which is located on the luminal surface of ductal epithelial cells, and extends more than 200-500 nm from the cell surface. The tumor-associated MUC1 antigens (polymorphic epithelial mucin, PEM) are high molecular weight glycoproteins (120-240 kDa and 400-420 kDa), with multiple highly branched carbohydrate side chains in O-glycosidic linkage to serine and threonine residues. The extracellular domain of the MUC1 glycoprotein contains a N-terminal region of 104 amino acids (aa), followed by the so-called VNTR region, containing a variable number of approximately 21 to 125 copies of a repetitive 20-amino acid sequence, which are arranged as tandem epeats, followed by a region of 228 aa at the C-terminal end of the extracellular domain of MUC1. The extracellular domain of MUC1 is linked by a transmembrane region of 28 aa to a short cytoplasmic domain of 72 aa at the C-terminal end of MUC1 (Gendler, 1988). In secreted forms of MUC1 the transmembran region and the cytoplasmatic region are deleted.

Seven different human mucins MUC1 to MUC7 are known, which posses an overall similar structure, with an N-terminal region followed by a VNTR region, containing tandem-repeat sequences. The tandem repeat sequences of the different mucins contain from 8 to 23 amino acids (MUC6 169 amino acids) and are rich in threonine, serine and proline residues. Human MUC1 is encoded by a hypervariable gene locus on chromosome 1 (region 21q) and the number of tandem repeats in the VNTR region varies as a result of genetic polymorphism depending on the length of the allele involved.

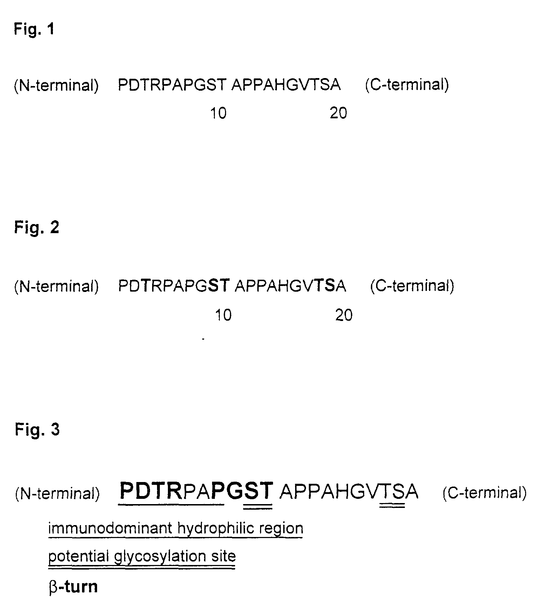

The 20-amino acid tandem repeat sequence of the VNTR region of MUC1 is shown in Figure 1. In Figure 1, the sequence Pro-Asp-Thr-Arg-Pro-Ala-Pro-Gly-Ser-Thr-Ala-Pro-Pro-Ala-His-Gly-Val-Thr-Ser-Ala is indicated for illustrative reasons in the shorter synonymous One-Letter Code of amino acids. However, the amino acid sequence indicated in Figure 1 is identical to the above-indicated sequence.

MUC1 is rich in O-linked glycosylation sites, because of the high content of serine (Ser, S) and threonine (Thr, T) residues of the 20-amino acid tandem repeat sequence shown in Figure 1. In each 20-amino acid tandem repeat sequence indicated in Figure 1, up to 5 serine or threonine residues can be glycosylated. In Figure 2, the potential glycosylation sites of the 20-amino acid tandem repeat sequence are indicated in bold. In Figure 2 amino acids are again indicated in One-Letter Code. Only 4 of the potential glycosylation sites indicated in Figure 2 have been recognised in the prior art as glycosylation sites of the MUC1 VNTR region, namely serine and threonine at positions 9 and 10 (S9, T10), and threonine and serine at positions 18 and 19 (T18, S19) in Figure 2 (J Taylor-Papadimitrou, 1991, Int J Cancer: 49, 1-5, P. 1 and table III). However, the fifth potential glycosylation site indicated in Figure 2, the threonine residue at position 3 (T3), is generally considered to be not glycosylated (J Taylor-Papadimitrou, 1991, Int J Cancer: 49, 1-5, P. 1 and table III).

In physiological fully glycosylated MUC1 the short sugar side chain at each of the glycosylation sites starts with N-acetylgalactosamine and galactose, forming the so-called core 1 structure, which is then extended by the addition of N-acetylglucosamine, fucose and sialic acid. In normal cells the glycosylation of newly synthesised peptides is a highly ordered process which involves as a first step the co-ordinated action of different glycosyltransferases (GalNAc-Transferases 1 to 4, Bennett-EP,1998).

In tumor cells a number of significant changes of MUC1 synthesis and glycosylation occur. These include:
a) loss of the polarised localisation of MUC1 restricted to the luminal side of the cell surface,
b) up-regulation (10 to 40-fold increase in breast carcinoma) of the expression of the MUC1 gene,
c) cytoplasmic accumulation and secretion of MUC1,
d) heterogeneity of the amino acids in the conserved tandem repeat domain in carcinoma cells (Muller-S, 1999),
e) aberrant glycosylation of MUC1, resulting in the appearance of novel carbohydrate epitopes and the unmasking of cryptic peptide epitopes; the aberrant glycosylation of MUC1 is a result of:
   1) altered glycosyltransferase activities yielding truncated carbohydrate side chains (T-antigen series and blood group related antigens of the Lewis series),
   2) increased activity of sialyltransferases.

In carcinoma cells the elongation of the core glycane is disturbed, leading to shorter and unbranched side chains which are terminated by sialic acid (Muller-S, 1999). This underglycosylated, heterogeneous carcinoma-associated MUC1 is formed in high amounts by adenocarcinomas of the breast, ovary, endometrium, lung, pancreas, kidney, bladder, prostate and other carcinomas and by at least two non cancerous tumours, namely multiple myeloma and erythroleukemia. Aberrantly glycosylated or underglycosylated MUC1 is therefore well suited as diagnostic and therapeutic tumor marker. "Aberrant glycosylation" or "underglycosylation" or "aberrant underglycosylation" of tumor-associated MUC1 antigen are used synonymously in this application.

Importantly, the epitope profile of MUC1 on malignant cells is clearly different from that of normal cells and therefore, tumor-associated underglycosylated MUC1 has immunogenic properties, i.e. it is recognised as antigen by the immune system and can elicit humoral and cellular immune responses. For example, tumor-associated MUC1 antigen can be recognised by HLA-restricted as well as non-MHC-restricted T cells (Finn, 1996). Several T-cell receptor specific (unglycosylated) peptide sequences from the VNTR and the N-terminal region of MUC1 were identified (Brugger, 1999). In addition, several different 9-mer peptides containing the APDTR epitope (comprising amino acids 20 and 1 to 4 of the sequence indicated in Figure 1) were shown to be targets of cytotoxic T lymphocytes (CTL), isolated from patients with breast, pancreatic and other cancers (overview by Finn et al., 1995). Cytotoxic T-cell precursors (CTLp) were identified in lymph nodes of breast cancer patients. In addition, several groups have demonstrated the existence of B cell-mediated immune responses in carcinoma patients. Human antibodies specific for underglycosylated MUC1 were documented in patients with ovarian carcinoma and breast cancer (Rughetti, A., et al., Cancer Res. 53 (1993) 2457-2461). An epitope within the 13-mer peptide TAPPAHGVTSAPD (comprising amino acids 10 to 20, 1 and 2 of the sequence indicated in Figure 1) is recognised by a human monoclonal antibody selected from a patient with ductal breast carcinoma (Kaul).

Tumor-associated MUC1 antigen is overexpressed in tissue samples of primary carcinomas and it can be identified in nearly all metastatic cells from these tumours. It is present on locoregional metastases, in visceral metastasis and most importantly on micrometastatic cells in different organs, such as lymph nodes, bone marrow, pleural effusions, ascites, and peripheral blood. In addition MUC1-overexpressing tumours are secreting large amounts of antigen into the circulation, where the antigen can be analysed as "tumor marker". Tumor-associated MUC1 antigen can therefore be detected in said tissue samples or in the body of a patient by immunological methods. This allows the development of immunological methods for the diagnosis and therapy of tumours, which carry tumor-associated MUC1 antigen.

Monoclonal antibodies (Mabs) specifically cross-reacting with tumor-associated MUC1 antigen can be used with great advantage to enhance the sensitivity and reliability of tumor diagnosis and to improve in vivo-based and ex vivo-based immune therapies. Such immunological methods depend on the specific reactivity of antibodies with the tumor-associated MUC1 antigen.

In the language of the invention the expressions "specifically cross-reacting", "specifically binding" and "(specifically) recognising" are used interchangeably, and refer to a specific non covalent interaction between an antigen and an antibody or between an antigen fragment bound to a major histocompatibility antigen complex (MHC) molecule of an antigen-presenting cell (APC) and a T cell receptor (TCR) of a cytotoxic T cell (CTL) or a T helper cell, as occurs in natural immune responses in the body, and as is well known and has in much detail been described in the fields of immunology and biochemistry.

In WO 99/40881 murine Mabs 7F11 and 1E4 have been described, which specifically bind to tumor-associated MUC1 antigen with unprecedented high specificity. Mabs 7F11 and 1E4 are unique in the prior art in regard to their specificity for tumor-associated underglycosylated MUC1 antigen. Mabs 7F11 and 1E4 do not cross-react with physiological fully glycosylated MUC1 protein or with unglycosylated synthetic MUC1 peptides. They are specific for tumor-associated underglycosylated MUC1 antigen, as produced and secreted for example by breast cancer cell lines T47-D and ZR75. Mabs 7F11 and 1E4 specifically bind to the carbohydrate structure of the aberrantly glycosylated 20-amino acid tandem repeat sequence of the VNTR region of the MUC1 antigen, produced by carcinoma cells.

Mabs 7F11 and 1E4 were produced as described in WO 99/40881, by procedures of the prior art. Mabs 7F11 and 1E4 were obtained by the immunisation of mice with a MUC1-containing fraction of a biochemical protein purification procedure of a mixture of ascites samples, obtained from a number of breast cancer patients. After the generation of hybridomas, the hybridoma clones were selected, which produce Mabs 7F11 and 1E4, which:
- cross-react with the MUC1-positive T47-D and ZR75 breast cancer cell lines, that produce aberrantly underglycosylated MUC1,
- do not cross-react with physiological fully glycosylated MUC1 from normal human breast milk,
- do not cross-react with the MUC1-negative LS 174T colon cancer cell line,
- do not cross-react with various (unglycosylated) synthetic peptides of MUC1, and
- retain their cross-reactivity with tumor-associated underglycosylated MUC1 antigen, if the antigen has been treated with neuraminidase or formalin, to remove N-terminal neuraminic acid residues.

From the above-described binding characteristics of Mabs 7F11 and 1E4 it is expected, that they recognize a carbohydrate-containing epitope of the tandem repeat sequence of the MUC1 VNTR region, that is highly distinctive for tumor-associated underglycosylated MUC1 antigen. No other monoclonal antibodies of the prior art possess comparable binding characteristics to the above-described binding specificity of Mabs 7F11 and 1 E4, that are unique in the prior art in regard to their specificity for tumor-associated underglycosylated MUC1 antigen. The knowledge of the epitope structure recognised by Mabs 7F11 and 1E4, is therefore of enormous importance for the development of novel diagnostic and therapeutic devices and methods.

The current development in the field of the invention is limited by the lacking availability of suitable tumor-related epitopes, so-called tumor markers, that occur with high specificity predominantly on tumor cells and that are, in addition, bound with high affinity by monoclonal antibodies. A large number of new strategies have been proposed in the prior art and are continuously being developed for novel immunological therapies of cancer. Strategies have been proposed to overcome the anergy of the immune system towards tumours in various ways, e.g. by enhancing the effectiveness of antigen presentation, using dendritic cells as antigen-presenting cells, by enhancing co-stimulation in various ways, by generating tumor specific effector cells, e.g. NK cells, CTL, T-helper cells; other novel therapies are based on the concept to carry cytotoxic compounds to tumor cells using carriers with high specificity for tumor cell-associated epitopes. All of the aforementioned novel strategies for the therapy of tumours, however, critically depend on the availability of epitopes that occur predominantly on tumor cells, i.e. that are highly specificity for tumor cells, and that are bound with high affinity by monoclonal antibodies (Mabs). A highly specific tumor epitope recognised by a strongly binding Mab can be used in any of the aforementioned novel therapies. However, success of the aforementioned therapies is limited by the quality of the epitope used as tumor marker, i.e. conferring specificity and affinity for the tumor to the active agents of said therapies. In fact, it has not yet been possible to use the aforementioned novel strategies with the expected success, because suitable tumor markers, i.e. highly specific tumor epitopes that are bound with high affinity by Mabs are missing in the prior art.

As in the above-mentioned examples of Mabs 7F11 and 1E4, most methods of the prior art for the production of monoclonal antibodies (Mabs) include an initial step of in vivo immunisation of an experimental animal. In addition, all known methods of the prior art for the production of Mabs include several steps of in vitro selection of hybridoma clones or other cellular clones, that produce Mabs with the desired binding specificity or cross-reactivity. An example of other cellular clones than hybridoma clones producing Mabs, are e.g. clones of genetically modified cells, expressing genes encoding antibodies or proteins with antibody-like activity (i.e. binding specificity). Both, the in vivo-immunisation and the selection of hybridomas or other cellular producer clones, critically depend on the availability of the antigen, that shall be specifically bound by the desired Mab.

With the methods of the prior art, however, there exists the problem, that the antigen that is recognised by said Mabs 7F11 and 1E4 has not been identified and is therefore not available. Therefore, further Mabs with the binding specificity of Mabs 7F11 and 1E4 can be produced only with an enormous effort, using the methods of the prior art, and success is not even certain. Thus, no reliable methods exist in the prior art, for the production of further Mabs with the same or comparable binding specificities as Mabs 7F11 or 1E4. In addition, in the prior art, there exist no methods for the production of antibody-like proteins or chimeric proteins containing antibodies or antibody-like structures in addition to parts of heterologous proteins, which can be obtained by molecular biological methods and which possess the same or comparable binding specificities as Mabs 7F11 or 1E4.

With the above-mentioned MUC1-containing fractions, that were originally used as immunogen in mice for the production of Mabs 7F11 and 1E4, there exists the problem, that the antigen that is recognised by Mabs 7F11 and 1E4 is not known. The original MUC1-containing fractions were purified from pooled material from different carcinoma patients. It is therefore unknown, how many different forms of tumor-associated MUC1 antigen were present in said original MUC1-containing fractions, the amino acid and carbohydrate structures of the different tumor-associated MUC1 antigens present in original MUC1-containing fractions are unknown, their concentrations are unknown, and finally the influence of different blood group related glycodeterminants in the said original fractions in not known and not reproducible. Thus, the antigen that was used for the production of Mabs 7F11 and 1E4 can not be isolated again from patients, because it can not be predicted from which patients samples should be taken, in order to obtain sufficient underglycosylated MUC1 antigen as source for the immunisation of mice for the generation of said antibody specifications.

The methods of the prior art for the production of Mabs 7F11 and 1 E4, comprising in vivo immunisation of experimental animals with an undefined mixture of tumor-associated MUC1 antigens, obtained from samples of tumor patients, and subsequent generation of immortal hybridomas, leads to the generation of a vast multitude of hybridomas, producing an enormous variety of Mabs with different binding specificities. The same holds true, if whole cells or partially purified glycoprotein fractions from carcinoma cells are used as immunogen. Fusions of the prior art lead to mixtures of hybridomas, producing an enormous variety of Mabs. In addition, it is frequently observed, that the immune response in mice and rats is dominated by clonotypes reactive with species-specific human HLA and blood group determinants. Hybridomas, producing Mabs with the desired binding specificity are therefore contained in said mixture of hybridomas in extremely low concentrations only, and may not even be present, at all.

The selection of hybridoma clones producing Mabs with the desired specific cross-reactivity to tumor-associated underglycosylated MUC1 antigen of Mabs 7F11 and 1E4, from the above-mentioned multitude of hybridomas is very laborious and time-consuming, and success cannot even be predicted. This is due to the lack of stringency of the selection procedures of the prior art, wherein the antigen that is recognised by said Mabs 7F11 and 1E4 is not available for the selection of hybridomas.

In addition, in the prior art, no procedures exist for the accurately defined generation of human B cells with specificity comparable to the above-mentioned Mabs 7F11 and 1E4. Likewise no procedure exists for the predictable generation of effector T cells, carrying T cell receptors (TCR), that cross-react with tumor-associated underglycosylated MUC1 antigen with comparable specificity as the above-mentioned Mabs 7F11 and 1E4. Effector T cells, specifically recognising tumor-associated underglycosylated MUC1 antigen could be used with enormous advantage in the therapy of tumours, especially but not limited to adjuvant tumor therapy and the treatment of minimal residual disease and the treatment of metastatic disease, by influencing and redirecting the cellular immune response of a patient against tumor cells producing tumor-associated underglycosylated MUC1 antigen.

In theory, effector T cell clones specifically cross-reacting with tumor-associated underglycosylated MUC1 antigen can be obtained by generating antigen-presenting cells (APC, including dendritic cells) "loaded with" or presenting tumor-associated MUC1 antigen and by ex vivo-stimulation of CD4-positive or CD8-positive T cells, obtained from the blood of a normal or tumor patient in the presence of suitable stimulatory cytokines, such as e.g. IL-2. However, methods for the generation of effector T cells by specific ex vivo stimulation, depend on the availability of a highly tumor specific antigen, such as a tumor-associated underglycosylated MUC1 antigen of the invention

As compared with the production of Mabs, additional factors inhibit the generation of effector T cells, specifically recognising tumor-associated MUC1 antigen. In order to prevent an immune response of the patient (or host) against the in vitro-generated effector T cells, syngeneic effector T cells, originating from the patient itself are most preferably required for transfer into the patient's body. Therefore, a quick and reliable method for the selection and generation of syngeneic effector T cells for individual patients is needed.

However, in contrast to the immortal hybridomas, used for the production of Mabs, the life span of T cells is limited and their handling in vitro is difficult. Thus, the time-consuming selection procedures of the prior art with uncertain success, using undefined mixtures of MUC1 antigen as in the above-described methods of the prior art for the selection of Mabs, cannot be applied in the generation of effector T cell clones.

The generation of effector T cells, specifically recognising underglycosylated tumor-associated MUC1 antigen is not possible in the prior art, because the epitopes of tumor-associated underglycosylated MUC1 antigen are unknown and hence are not available for the ex vivo-stimulation of T cells. In the prior art, only undefined MUC1 antigen fractions purified from tumor cells (Bastert unpublished), and (unglycosylated) synthetic peptides of the MUC1 VNTR region ( Finn, 1996, Reddish,1998) and STn-KLH glyconeoconjugates (Longenecker, 1993, McLean, 1996) have been used in immunotherapeutical trials. Neither the aforementioned undefined mixtures of different MUC1 antigens, nor synthetic peptides of the MUC1 VNTR region or STn vaccines have been used in the prior art with success for the generation of human effector T cells, that recognize tumor-associated MUC1 antigen with a comparably high specificity as Mabs 7F11 and 1E4.

In the prior art, no methods exist for the production of further Mabs like the above-mentioned Mabs 7F11 and 1E4, which cross-react with tumor-associated underglycosylated MUC1 antigen with unique specificity, for the production of antibody-like recombinant proteins and for the generation of B cells, plasma cells and effector T cells with a comparably high specificity for tumor-associated underglycosylated MUC1 antigen as Mabs 7F11 and 1E4, because the MUC1 antigen that is recognised by said Mabs 7F11 and 1E4 is not available, and the structure of its epitope that is recognised by said Mabs 7F11 and 1 E4 is not known.

In the prior art, a number of peptide, carbohydrate and cryptic peptide epitopes have been characterised on tumor-associated MUC1 antigen. However, unexpectedly, no carbohydrate-containing epitopes of tumor-associated underglycosylated MUC1 antigen could be characterised (Taylor-Papadimitriou, 1990, Int J Cancer, 49, 1-5; Apostolopoulos and Mc Kenzie, 1994, Critical Reviews in Immunology, 14(3&4), 293-309; Price et al., 1998, Tumor Biol, 19 (suppl 1), 1-20). The majority of Mabs of the prior art against tumor-associated MUC1 antigen specifically bind to (unglycosylated) peptide epitopes within the hydrophilic amino acid sequence PDTRPAP (amino acids 1 to 7 of the sequence indicated in Figure 1) of the MUC1 VNTR repetitive sequence. However, none of the aforementioned Mabs possesses the high binding specificity for tumor-associated MUC1 antigen of Mabs 7F11 or 1E4.

The putative secondary structure of the tandem repeat sequence (indicated in Figure 1) of the MUC1 VNTR region is schematically represented in Figure 3. According to hydrophobicity profiles and analyses of secondary structure, the tandem repeat sequence contains a hydrophilic region extending through residues Pro-Asp-Thr-Arg-Pro-Ala-Pro (residues 1 to 7 of the sequence indicated in Figure 1; in One-Letter Code: PDTRPAP; indicated by simple underlinement in Fig. 3). The hydrophilic PDTRPAP region is identical to the immunodominant region of the MUC1 VNTR tandem repeat sequence, that is specifically bound by the majority of the above-mentioned peptide specific Mabs of the prior art. The immunodominant hydrophilic PDTRPAP region is flanked by two potential glycosylation sites, encompassing Thr 18 (T18), Ser 19 (S19) and Ser 9 (S9), Thr 10 (T10), respectively, of the 20-amino acid tandem repeat sequence indicated in Figure 1 (indicated by double underlinement in Fig. 3).

According to the high proline (25%), glycine, serine and glutamine content, the MUC1 VNTR forms proline β-turn helixes (Matsushima, 1990) consistent with the observed formation of extended rod-shaped structures (Hilgers). Thus, the immunodominant hydrophilic PDTRPAP sequence is thought to contain two β-turn secondary structure elements at its N-terminal and C-terminal flanking sequences Pro-Asp-Thr-Arg (residues 1 to 4 of the sequence indicated in Figure 1; in One-Letter Code: PDTR) and Pro-Gly-Ser-Thr (residues 7 to 10 of the sequence indicated in Figure 1; in One-Letter Code: PGST), respectively (indicated in bold letters in Fig. 3). The rest of the tandem repeat sequence is hydrophobic and essentially lacking secondary structure.

The immunodominant hydrophilic PDTRPAP region of the tandem repeat sequence of the MUC1 VNTR is flanked by two β-turns and is thought to be at the surface of the mucin molecule and therefore accessible for Mabs. However, only Mabs directed against peptide epitopes within this region could be obtained by immunisation of mice with tumor-associated MUC1 antigen (V Apostolopoulos and IFC McKenzie, 1994, Critical Reviews in Immunology, 14(3&4), 293-309, p. 300; Price et al. 1998, Tumor Biol, 19 (suppl 1), 1-20).

The majority of known epitopes (> 35 according to the results of the TD4-Workshop on MUC1) of tumor-associated MUC1 antigen are peptide epitopes. In the prior art, several Mabs with specificity for carbohydrate-containing/associated epitopes of tumor-associated MUC1 antigen are described. One group of carbohydrate specific antibodies, including M26, 115D8, 3E1.2, 5F4, MA695, FH6, are dependent on terminal sialic acid (Hilgers, Galanina, 1998) and therefore distinct from 7F11 and 1E4. Other Mabs including A78-G/A7, SH1, DH1, FH6, M26 and BW835 are reactive with more complex blood group related antigens, such as tetrasaccharid (SiaLe^{x}, SiaLeₐ), trisaccharide (Le^{x}, Le^{a}, Aₜᵣᵢ, Bₜᵣᵢ₎ and disaccharide TF, SiaTn, LactNAc antigens, which are also distinct from 7F11 and 1E4 epitopes. Finally, for several Mabs (27.1, KC4, 7539MR) the carbohydrate structure is completely unknown.

An enormous number of possible tumor-associated carbohydrate-containing or carbohydrate-modulated epitopes of MUC1 antigen can be expected from the numerous possible alterations of the carbohydrate side chains and in combination thereof, from the known peptide sequence heterogeneity of the VNTR region. Alterations of the carbohydrate side chains can firstly occur at any of the five potential glycosylation sites indicated in Figure 2. Secondly, it can lead to the loss of any terminal sugar, thirdly to modification or loss of the backbone glycane structure and finally to modification and partial or complete loss of the core glycans galactose and N-acetylgalactosamine. It has been observed, however, that generally, premature termination of the glycan structure in tumor cells seems to be associated with the overexpression and enhanced activity of sialytransferases (Hilgers, 1998).

Because of the enormous number of possible structural variants of tumor-associated underglycosylated MUC1 antigen, the structure of the epitopes, that are specifically bound by Mabs 7F11 and 1E4, cannot be predicted.

A first part of the object of the invention is, to devise the minimum structures of the immunogenic epitopes of tumor-associated underglycosylated MUC1 antigen, that are specifically and with high affinity bound by Mabs 7F11 and 1E4.

A second part of the object of the invention is to enable the easy, fast and reliable production of further Mabs with the binding specificities of Mabs 7F11 and 1E4 for tumor-associated underglycosylated MUC1 antigen.

A third part of the object of the invention is to enable the easy, fast and reliable production of antibody fragments, of mabs or antibody-like proteins produced with molecular biological methods and of chimeric proteins carrying antibodies or antibody fragments linked to heterologous proteins, comprising antibodies or antibody-like structures with the binding specificity of Mabs 7F11 and 1E4 for tumor-associated underglycosylated MUC1 antigen.

A fourth part of the object of the invention is, to provide methods for the generation of B cells, effector NK cell clones and effector T cell clones, which cross-react with the same or with comparable specificity with tumor-associated unglycosylated MUC1 antigen as Mabs 7F11 and 1E4.

A fifth part of the object of'the invention is the use of the immunologically active proteins or effector T or NK cell clones of the invention for the diagnosis and therapy of tumours carrying tumor-associated underglycosylated MUC1 antigen, that is specifically bound by said immunologically active proteins or effector T cells of the invention.

A sixth part of the object of the invention is a method for the active immunisation of tumor patients and healthy individuals, using the epitopes of the invention.

The object of the invention is solved by the features of the characterising part of independent claim 1. Preferred embodiments of the invention are contained in dependant claims 2 to 15.

Figures 1 to 3 illustrate the prior art, Figure 4a shows the embodiment of the invention according to independent claim 1, Figures 4b to 4f show preferred embodiments of the invention, and Figures 5 and 6 further illustrate the invention.

### Figures

- Figure 1: shows the 20-amino acid tandem repeat sequence of the VNTR region of human MUC1 protein.
- Figure 2: shows the potential glycosylation sites of the sequence shown in Figure 1.
- Figure 3: shows the putative secondary structure elements of the sequence shown in Figure 1
- Figure 4a: shows the glycopeptide epitope of the invention according to Sequence No. 1.
- Figure 4b: shows the glycopeptide epitope of the invention according to Sequence No. 2.
- Figure 4c: shows the glycopeptide epitope of the invention according to Sequence No. 3.
- Figure 4d: shows the glycopeptide epitope of the invention according to Sequence No. 4.
- Figure 4e: shows the glycopeptide epitope of the invention according to Sequence No. 5.
- Figure 4f: shows the glycopeptide epitope of the invention according to Sequence No. 6.
- Figure 5: shows a pepscan analysis of the glycopeptide epitopes of the MUC1 VNTR.
- Figure 6: shows the structure of a 13-mer glycopeptide comprising amino acids 18, 19, 20 and 1 to 10 of the sequence shown in Figure 1.

In the following, the invention and preferred embodiments of the invention are described. The invention provides the structure and the uses of the glycopeptide epitopes of tumor-associated underglycosylated MUC1 antigen that are bound by Mabs 7F11 and 1E4, which were disclosed in the International Patent Application 99/40881.

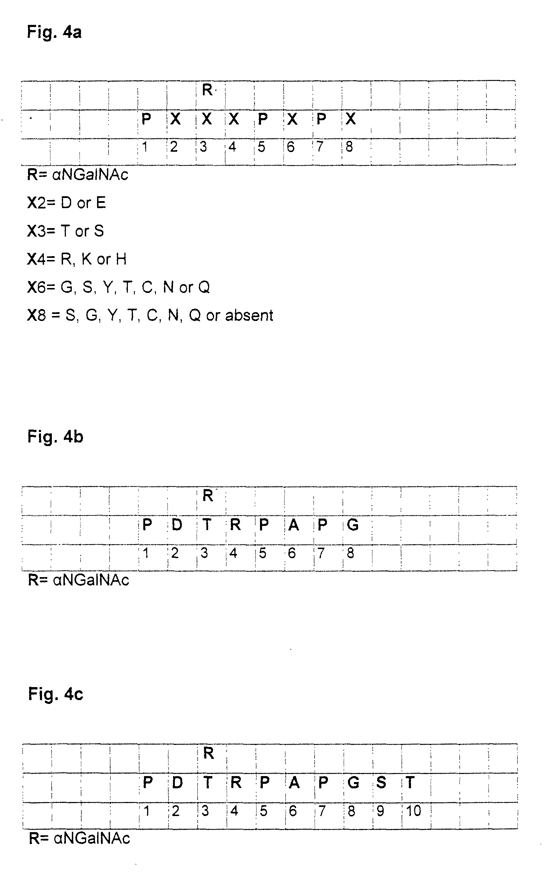

In the following, the epitopes of the invention of tumor-associated underglycosylated MUC1 antigen that are specifically bound by Mabs 7F11 and 1E4 are described. The minimal epitope of the invention is the epitope indicated in Sequence No. 1. Sequences No. 2 to 6 indicate preferred embodiments of the epitopes of the invention. The structures of the epitopes of the invention of Sequences No. 1 to 6 are shown in Figures 4a to 4f, respectively. In Figures 4a to 4f, the relative positions of Sequences No. 1 to No. 6 according to the numbering of the 20-amino acid tandem repeat sequence of the MUC1 VNTR region shown in Figure 1, are in addition indicated.

The tumor-associated underglycosylated MUC1 antigens that are bound by Mabs 7F11 and/or 1E4 are selected from the group of glycopeptides and glycoproteins comprising at least one 8-mer glycopeptide epitope of the invention according to Sequence No. 1, wherein the amino acid residue at position 3 of Sequence No. 1 carries at a single N-acetylgalactosamine molecule (αGalNAc) or a single N-acetylglucosamine molecule (αGluNAc) in O-glycosidic linkage. The epitopes of the invention according to Sequence No. 1 comprise in addition proline residues at positions 1, 5 and 7, a negatively charged amino acid residue at position 2, a positively charged amino acid residue at position 4, a polar uncharged amino acid residue at position 6 and a polar uncharged amino acid residue at position 8. The amino acid residue at position 8 can in addition be missing in the epitopes of the invention according to Sequence No. 1, in particular in the epitopes of the invention which are bound by Mab 1E4.

In Figure 4a the 8-mer glycopeptide epitopes of Sequence No. 1 of the invention, which are bound by Mabs 7F11 and 1E4 are indicated in the One-Letter Code of amino acids. The numbers in Figure 4a indicate in addition the numbering of the 20-amino acid tandem repeat sequence of the MUC1 VNTR, shown in Figure 1. "R" indicates the single carbohydrate (αGalNAc or αGluNAc) molecule that is linked in O-glycosidic linkage to the threonine or serine residue at position 3 of Sequence No. 1. "P" indicates the proline residues at positions 1, 5 and 7 of Sequence No. 1. "X2" indicates the aspartic acid or glutamic acid residue at position 2 of Sequence No. 1. "X3" indicates the threonine or serine residue at position 3 of Sequence No. 1, which carries a single N-acetylgalactosamine molecule (αGalNAc) or a single N-acetylglucosamine molecule (αGluNAc) in O-glycosidic linkage. "X4" indicates the arginine, lysine or histidine residue at position 4 of Sequence No. 1. "X6" indicates the glycine, serine, tyrosine, threonine, cysteine, asparagine or glutamine residue at position 6 of Sequence No. 1. "X8" indicates the serine, glycine, tyrosine, threonine, cysteine, asparagine or glutamine residue at position 8 of Sequence No. 1. The amino acid residue at position 8 of Sequence No. 1 can be missing in the epitopes of the invention, in particular in the epitopes of the invention which are bound by Mab 1E4.

According to a first preferred embodiment of the invention, the epitope that is bound by Mab 7F11 and/or 1E4 comprises the 8-mer glycopeptide of Sequence No. 2, wherein the threonine residue at position 3 of Sequence No. 2 carries at a single N-acetylgalactosamine molecule (αGalNAc) in O-glycosidic linkage. The epitopes of the invention according to Sequence No. 2 comprise in addition proline residues at positions 1, 5 and 7, an aspartic acid residue at position 2, an arginine residue at position 4, a glycine residue at position 6 and a serine residue at position 8. The epitopes of the invention according to Sequence No. 2 may in addition lack the serine residue at position 8, in particular in the epitopes of the invention which are bound by Mab 1E4.

In Figure 4b the 8-mer glycopeptide epitope of Sequence No. 2 of the invention, which is bound by Mabs 7F11 and 1E4 is indicated in the One-Letter Code of amino acids. The numbers in Figure 4a indicate in addition the numbering of amino acids according to the MUC1 VNTR tandem repeat sequence indicated in Figure 1. The letter "R" indicates the position of the single αGalNAc molecule linked in O-glycosidic linkage to the threonine residue at position 3 of Sequence No. 2.

The 8-mer glycopeptides of the invention of Sequences No. 1 and No. 2, that are specifically bound by Mabs 7F11 and 1E4, are the first known tumor specific MUC1-associated Tnα-glycoepitopes that are bound by a Mab with specificity for a carbohydrate moiety and in addition with strict peptide sequence specificity.

The epitopes of the invention may contain additional amino acids on the amino- and/or carboxy-terminal side of Sequences No 1 and 2. Further epitopes of the invention that specifically cross-react with Mabs 7F11 and 1E4 and that are based on Sequence No. 2 are indicated in Sequence No. 3, Sequence No. 4, Sequence No. 5 and Sequence No. 6. The invention is, however, in no respect limited to the epitopes described in Sequences No. 1 to 6. The invention includes the glycopeptides and glycoproteins comprising any amino acid sequence, that are bound by at least one of Mabs 7F11 or 1E4 and that in addition comprise any of Sequences No. 1 to 6 of the invention with any of the amino acid substitutions at positions 2, 3, 4, 6 and 8 of Sequence No. 1, wherein at least one amino acid residue is glycosylated, namely the threonine or serine residue at position 3 of Sequence No. 1 carries a single N-acetylgalactosamine molecule (αGalNAc) or a single N-acetylglucosamine molecule (αGluNAc) in O-glycosidic linkage.

According to a preferred embodiment of the invention, the epitope that is bound by Mab 7F11 and 1E4 comprises the 10-mer glycopeptide of Sequence No. 3, wherein the threonine residue at position 3 of Sequence No. 3 is glycosylated, carrying a single N-acetylgalactosamine molecule (αGalNAc) in O-glycosidic linkage. In Figure 4c the 10-mer glycopeptide epitope of Sequence No. 3 is indicated the One-Letter Code of amino acids. The numbers in Figure 4c indicate in addition the numbering of amino acids according to the MUC1 VNTR tandem repeat sequence indicated in Figure 1. The letter "R" indicates the position of the αGalNAc moiety. The epitopes of the invention also comprise any variants of the glycopeptide of Sequence No. 3, that are in addition glycosylated at the serine residue at position 9 and/or the threonine residue at position 10 of Sequence No. 3. The aforementioned additional glycosylation sites of Sequence No. 3 may comprise carbohydrate structures of any structure, in particular single αGalNAc molecules in O-glycosidic linkage.

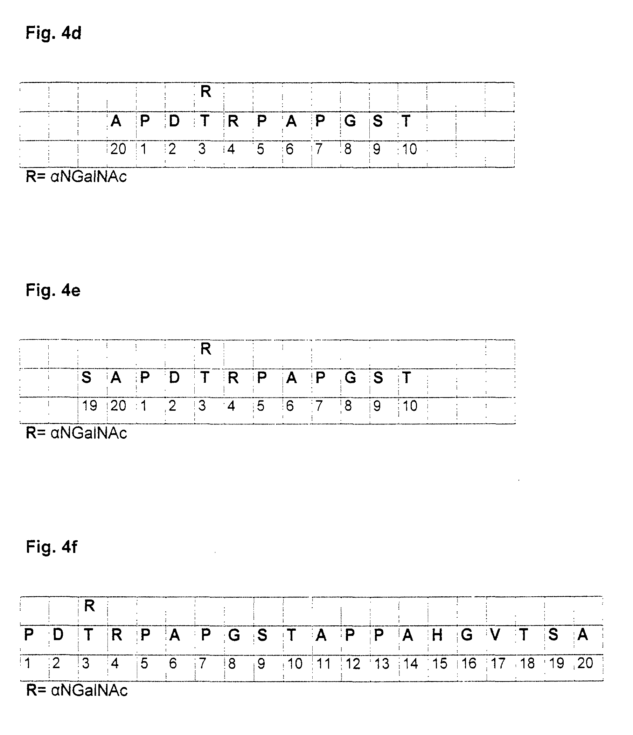

The 11-mer epitope of the invention of Sequence No. 4, that is specifically bound by Mabs 7F11 and 1E4, comprises amino acids 20 and 1 to 10 of the MUC1 VNTR tandem repeat sequence indicated in Figure 1, wherein the threonine residue at position 4 of Sequence No. 4 is glycosylated, carrying a single N-acetylgalactosamine molecule (αGalNAc) in O-glycosidic linkage. In Figure 4d the 11-mer glycopeptide epitope of Sequence No. 4 is indicated in the One-Letter Code of amino acids. The numbers in Figure 4d indicate in addition the numbering of amino acids according to the MUC1 VNTR tandem repeat sequence indicated in Figure 1. The letter "R" indicates the position of the αGalNAc moiety at position 4 of Sequence No. 4 (indicated in Figure 4d by number 3, according to the numbering of the MUC1 VNTR tandem repeat sequence indicated in Figure 1). The epitopes of the invention also comprise any variants of the glycopeptide of Sequence No. 4, that are in addition glycosylated at the serine residue at position 10 and/or the threonine residue at position 11 of Sequence No. 4 (indicated in Figure 4d by numbers 9 and 10, respectively, according to the numbering of the MUC1 VNTR tandem repeat sequence indicated in Figure 1). The aforementioned additional glycosylation sites of Sequence No. 4 may comprise carbohydrate structures of any structure, in particular single αGalNAc molecules in O-glycosidic linkage.

The 12-mer epitope of Sequence No. 5 comprises amino acids 19, 20 and 1 to 8 of the MUC1 VNTR tandem repeat sequence indicated in Figure 1, wherein the threonine residue at position 5 of Sequence No. 5 is glycosylated, carrying a single N-acetylgalactosamine molecule (αGalNAc), preferably in O-glycosidic linkage. In Figure 4e the 12-mer glycopeptide epitope of Sequence No. 5 is indicated in the One-Letter Code of amino acids. The numbers in Figure 4e indicate in addition the numbering of amino acids according to the MUC1 VNTR tandem repeat sequence indicated in Figure 1. The letter "R" indicates the position of the αGalNAc moiety. The epitopes of the invention also comprise any variants of the glycopeptide of Sequence No. 5, that are in addition glycosylated at the serine residue at position 1 of Sequence No. 5, the serine residue at position 11 of Sequence No.5 and the threonine residue at position 12 of Sequence No. 5 (indicated in Figure 4e by numbers 19, 9 and 10, respectively, according to the numbering of the MUC1 VNTR tandem repeat sequence indicated in Figure 1). The aforementioned additional glycosylation sites of Sequence No. 5 may comprise any carbohydrate structures, in particular single αGalNAc molecules in O-glycosidic linkage.

The 20-mer epitope of Sequence No. 6 comprises amino acids 1 to 20 of the MUC1 VNTR tandem repeat sequence indicated in Figure 1, wherein the threonine residue at position 3 of Sequence No. 6 is glycosylated, carrying a single N-acetylgalactosamine molecule (αGalNAc) in O-glycosidic linkage. In Figure 4f the 20-mer glycopeptide epitope of Sequence No. 6 is indicated in the One-Letter Code of amino acids. The numbers in Figure 4f indicate in addition the identical numbering of amino acids according to the MUC1 VNTR tandem repeat sequence indicated in Figure 1. The letter "R" indicates the position of the αGalNAc moiety. The epitopes of the invention also comprise any variants of the glycopeptide of Sequence No. 6, that are in addition glycosylated at the serine residue at position 9 and/or the threonine residue at position 10 and/or the threonine residue at position 18 and/or the serine residue at position 19 of Sequence No. 6. The aforementioned additional glycosylation sites of Sequence No. 6 may comprise carbohydrate structures of any structure, in particular single αGalNAc molecules in O-glycosidic linkage.

The antigens of the invention that are bound by Mab 7F11 and/or 1E4 comprise in addition the epitopes of the invention that contain additional amino acids on the amino- and/or carboxy-terminal side of any of Sequences No 1 to No. 6, that result from the arrangement of multiple copies of the 20-amino acid sequence indicated in Figure 1 in the form of tandem repeats.

The epitopes of the invention in addition comprise any glycopeptide or are contained in any glycoprotein, comprising at least one glycopeptide of Sequence No. 2, comprising any amino acid substitutions, wherein:
- alanine, valine, leucine, isoleucine, proline, tryptophane, phenylalanine and methionine are substituted for each other; and/or
- glycine, serine, tyrosine, threonine, cysteine, asparagine and glutamine are substituted for each other; and/or
- aspartic acid and glutamic acid are substituted for each other; and/or
- lysine, arginine and histidine are substituted for each other; and/or
- glycine and alanine are substituted for each other.

In the following, an example of a determination of the structure of an epitope of the invention is described. The example illustrates, how the amino acid sequence of an epitope of the invention and the number, position and identity of the glycosylated threonine or serine residues carrying a single αGalNAc or αGluNAc residue in O-glycosidic linkage can be determined.

The invention is in no respect limited to the epitopes specifically bound by Mabs 7F11 and/or 1E4 that can be identified, using the method of the example. However, the invention comprises all epitopes bound by Mabs 7F11 and 1E4 that are structurally similar to the epitopes of Sequence No. 1 to 6 and comprise the threonine or serine residue at the relative position indicated in any of Sequences No. 1 to No. 6 that carries a single αGalNAc or αGluNAc molecule in O-glycosidic linkage, that can be identified by the skilled in the art using similar methods as in the following example.

In addition, the skilled in the art can use similar methods as in the following example to identify Mabs of the invention, recombinant antibodies of the invention, antibody-like proteins of the invention containing antibodies or active parts of antibodies, e.g. complemeritarity-determining regions, and chimeric proteins of the invention containing antibodies or active parts of antibodies and in addition heterologous proteins or parts thereof, that have been generated, using the epitopes of the invention. In such an analysis, binding of Mab 7F11 or 1E4 to an epitope of the invention is used as standard.

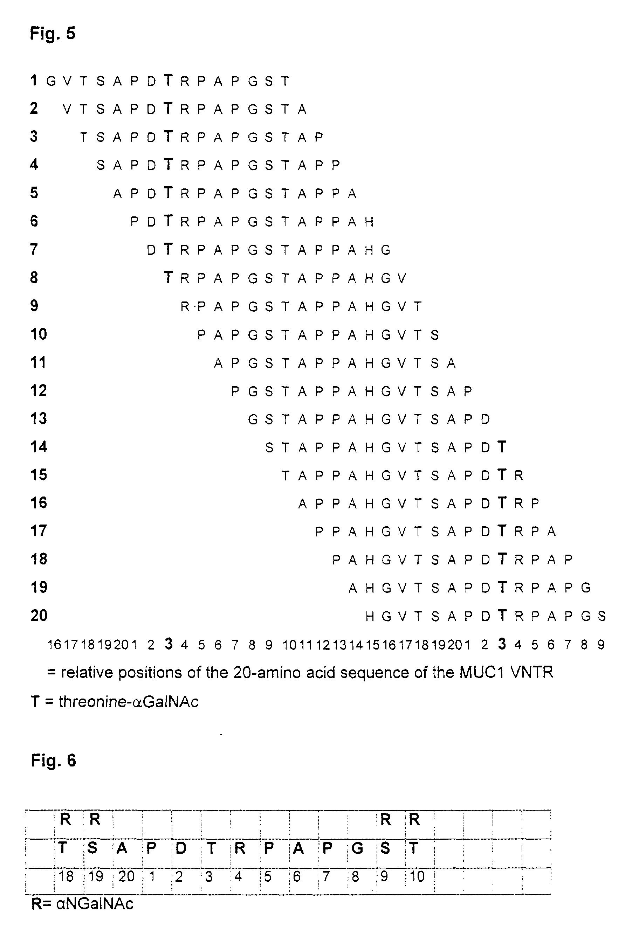

The binding of Mabs 7F11 and 1E4 to the epitopes of the invention and in particular to the glycopeptide epitope of Sequence No. 2 was verified by epitope mapping, as follows. Figure 5 is a schematic representation of a pepscan analysis of the epitopes of the invention, using Mab 7F11. A series of 20 partially overlapping 15-mer MUC1 glycopeptides was synthesised, wherein each glycopeptide overlapped its preceding neighbour in the series in all but one lacking amino acid at its N-terminus and contained one additional amino acid at its C-terminus. 15-mer MUC1 glycopeptides were synthesised with the Fmoc chemistry (Nastainczyk, 1995), wherein serine and threonine can be introduced as Fmoc-T- or S-(GalNAc(Ac)l-f-D)-OH derivatives (Bachem, Heidelberg, FRG). Glycopeptides were tethered at their carboxy-terminus to the tips of polyethylene pins or onto discrete spots on solid membranes (Geysen, 1987). Membrane stripes were prepared that contained 20 discrete spots, each spot comprising a single type of glycopeptide of the above-mentioned series of 20 partially overlapping 15-mer glycopeptides.

At the left border of Figure 5, individual 15-mer MUC1 glycopeptides of the above-mentioned series of 20 partially overlapping 15-mer glycopeptides are numbered from 1 to 20. In Figure 5, the amino acid sequences of the 15-mer glycopeptides are indicated in One-Letter Code. The relative positions indicated at the bottom of Figure 5 indicate the relative positions of amino acids within the series of partially overlapping 15-mer glycopeptides, according to the numbering of amino acids in the MUC1 VNTR tandem repeat sequence indicated in Figure 1. Thus, relative positions 1 to 20 indicated at the bottom of Figure 5 correspond to amino acids 1 to 20 of the MUC1 VNTR tandem repeat sequence indicated in Figure 1.

In Figure 5, the threonine residues at relative position 3 are marked in bold. They correspond to the threonine residue at position 3 of the MUC1 VNTR tandem repeat sequence indicated in Figure 1. The bold marked threonine residues in Figure 5 were replaced by the Fmoc-T-(GalNAc(Ac)l-f-D)-OH derivative, and therefore carried a single αGalNAc molecule in O-glycosidic linkage. Thus, the bold marked threonine residues in Figure 5 corresponded to the glycosylated threonine residue at position 3 of Sequences No. 1, 2, 3 and 6, at position 4 of Sequence No. 4 and at position 5 of Sequence 5 of the epitopes of the invention.

Individual Pepscan membrane stripes, containing 20 spots with the above-mentioned partially overlapping 15-mer glycopeptides, were probed with different Mabs for specific binding of glycopeptides as follows: Pepscan stripes were blocked with PBS, 1% BSA and incubated for one hour at room temperature on a shaker with Mabs at a concentration 200 ng/ml in PBS, 1% BSA and 0.05% Tween 20. Stripes were washed for 10 min with PBS, 0.05% Tween20. Mabs were detected by goat-anti-mouse IgG-alkaline phosphatase conjugates diluted 1:20 000 (Dianova, Hamburg, FRG) and the substrate BCIP/NBT (BioRad, München, FRG).

The results from an individual pepscan experiment using Mab 7F11 are indicated in Figure 5. The results of the experiment indicated in Figure 5 represent only a single example of an experiment that was repeated many times and was modified in a variety of ways, to help to determine the glycopeptide epitopes of the invention for Mabs 7F11 and 1E4.

In the individual experiment depicted in Figure 5, Mab 7F11 bound strongly to glycopeptides 2, 3, 4, 19 and 20 and bound weakly to glycopeptides 1 and 5. The amino acids shared by glycopeptides 1 to 5 and by glycopeptides 19 and 20 are underlined. Glycopeptides weakly cross-reacting with Mab 7F11 are indicated by single underlinement and glycopeptides strongly cross-reacting with Mab 7F11 are indicated by double underlinement.

The minimal epitope for Mab 7F11 that could be determined in the individual experiment represented in Figure 5 corresponds to the overlapping part of glycopeptides 2, 3, 4, 19 and 20, namely to residues 19, 20 and 1 to 8 - in N-terminal to C-terminal direction - of the MUC1 VNTR tandem repeat sequence indicated in Figure 1, wherein the threonine residue at position 3 according to the sequence indicated in Figure 1 carried a single αGalNAc molecule in O-glycosidic linkage. In further experiments, Mab 7F11 strongly cross-reacted also with glycopeptide 1 depicted in Figure 5, thereby confirming the above-mentioned minimal epitope. Similar results were obtained in comparable experiments using Mab 1E4. In addition, in further comparable experiments significant binding of Mabs 7F11 and 1E4 to glycopeptides 5 and 6 depicted in Figure 5, was observed, thereby confirming the glycopeptide epitope of the invention of Sequence No. 2, which comprises amino acids 1 to 8 of the MUC1 VNTR tandem repeat sequence indicated in Figure 1. In control analyses it was observed that unglycosylated peptides comprising the amino acid sequences of any of the glycopeptides shown in Figure 5 were not bound by Mabs 7F11 or 1E4.

In similar experiments, a difference between the minimal epitope required for binding of Mab 1E4 and Mab 7F11 was observed. Mab 1E4 binds to a glycopeptide that is lacking the C-terminal glycine residue of Sequence No. 2 and comprises only amino acids 1 to 7 of Sequence No. 2, with comparable affinity as it binds to a glycopeptide comprising Sequence No. 2. Therefore the C-terminal glycine residue of Sequence No. 2 is not necessary for the binding of Mab 1E4.

Sequence No. 2 comprises the minimal epitope of the invention that is bound by Mabs 7F11 and 1E4, in the sense, that the epitope that is bound by Mabs 7F11 and 1E4 need not comprise additional structural features, further than are comprised in the glycopeptide of Sequence No. 2. In particular, glycosylation of the threonine residue at position 3 of the MUC1 VNTR tandem repeat sequence indicated in Figure 1 with a single αGalNAc residue, is sufficient to mediate the carbohydrate-dependent binding of Mabs 7F11 and 1E4. In addition, binding of Mabs 7F11 and 1E4 to their epitopes depends strictly on the amino acid sequence of the epitope. Examples of glycopeptides of the invention, that comprise suitable amino acid sequences for the binding of Mabs 7F11 and 1 E4, are given by Sequences No. 2 to 6. For the binding of Mab 1E4, Sequence No. 2 needs only to comprise amino acids 1 to 7.

The epitopes of the invention that are specifically bound by Mabs 7F11 and 1E4, comprise at least the structural features of Sequence No. 1. They comprise amino acids 1 to 8 of Sequence No. 1 (at least amino acids 1 to 7 of Sequence No. 1 for Mab 1E4), the threonine or serine residue at position 3 of Sequence No. 1 carrying a single αGalNAc or αGluNAc residue in O-glycosidic linkage, the proline residues at positions 1, 5 and 7 of Sequence No. 1 that probably determine β-turn secondary structure elements, the acidic amino acid residue at position 2 of Sequence No. 1 and the basic amino acid residue at position 4 of Sequence No. 1 the flank the glycosylated residue on either side, the polar uncharged amino acid residues at position 6 of Sequence No. 1 between the second and third proline residue and the polar uncharged amino acid residue at position 8 of Sequence No. 1.

As illustrated in the example of Figure 5, pepscan analyses allow to verify the boundaries of the minimal glycopeptide epitopes of the invention and allow to verify the carbohydrate structure of the glycopeptide epitopes of the invention, by assaying for the binding of Mabs 7F11 and 1E4. The invention therefore comprises structural variants of the epitopes of the invention, in particular the epitopes of Sequence No. 1, that can be found by the experienced in the art by carrying through pepscan analyses with structural variants of Sequence No. 2, in particular with variants containing fewer amino acid residues or containing single or few amino acid substitutions. Said amino acid substitutions comprise in particular so-called "conservative" substitutions by amino acids with similar properties, such as similar size, similar charge similar polarity, or similar hydrophobicity.

Figure 6 indicates the structure of a 13-mer glycopeptide of the MUC1 VNTR, comprising amino acids 18, 19, 20 and 1 to 10 of the MUC1 VNTR tandem repeat sequence indicated in Figure 1. The numbers in Figure 6 indicate the numbering of amino acids according to the sequence indicated in Figure 1. In the MUC1 VNTR region, the partial sequence shown in Figure 6 occurs as a result of the tandem repeat arrangement of the sequence indicated in Figure 1. The glycopeptide of Figure 6 comprises all five potential glycosylation sites of the MUC1 VNTR tandem repeat sequence indicated in Figure 1. The dimeric glycosylation sites, comprising amino acids 18, 19 and 9, 10 of the sequence indicated in Figure 1 are arranged at the amino and carboxy terminal end of the glycopeptide of Figure 6, respectively. The dimeric glycosylation sites of the glycopeptide of Figure 6 carry single αGalNAc residues at each of the four individual glycosylation sites. However, the fifth potential glycosylation site, the threonine residue at position 3 of the MUC1 VNTR tandem repeat sequence indicated in Figure 1, is not glycosylated in the glycopeptide shown in Figure 6. The minimal epitope of the invention, namely the glycopeptide according to Sequence No. 2, is therefore only present in unglycosylated form in the glycopeptide shown in Figure 6 and is flanked by the above-mentioned dimeric glycosylation sites at its amino and carboxy-terminal ends, which carry single αGalNAc residues at each of the four individual glycosylation sites.

The 13-mer glycopeptide depicted in Figure 6 is not bound by Mabs 7F11 and 1E4. Thus, the single αGalNAc moiety of threonine 3 in Sequence No. 2 is essential for the binding of Mabs 7F11 and 1E4 and cannot be substituted by glycosylation with single αGalNAc residues of any of the four other potential glycosylation sites of Sequence No. 1, flanking the epitope of Sequence No. 2 at its N-terminal and C-terminal ends within the MUC1 VNTR.

The glycopeptide shown in Figure 6 is completely negative with Mabs 7F11 and 1E4. Therefore, Tn and STn substitutions at adjacent serine or threonine residues which are epitopes of the TAG72 mucin antibodies B72.3 and CC49 (Reddish, MA, 1997) of the prior art, and which are the main structural components of the synthetic carbohydrate vaccine Theratope® developed by Biomira (Longenecker, 1996, and Biomira Inc., Edmonton, Can) are clearly different from the glycopeptide epitopes of the invention.

In the following, binding of Mabs 7F11 and 1E4 to the epitopes of the invention is compared to the binding of several Mabs of the prior art to the epitopes of the invention. The results of determinations of the specificities and affinities (binding strength) with which said Mabs bind to the epitopes of the invention and to control antigens are indicated in Table 1 and Table 2, respectively.

The data indicated in Table 1 and 2 represent the results of single experiments, only. Therefore, the invention is in no respect limited to the specificities and to the affinities indicated in Table 1 and 2, respectively, with which the epitopes of the invention were bound by Mabs 7F11 and/or 1E4 in said single experiments. However, similar differences between the binding of Mabs 7F11, 1E4 and the indicated antibodies of the prior art to the epitopes of the invention, were observed in further experiments.

The binding specificities and binding affinities described herein, illustrate the differences between the epitopes of the invention and the epitopes bound by several well-known Mabs of the prior art. In addition, the skilled in the art can identify Mabs of the invention that bind to the epitopes of the invention in similar experiments. In particular, Mabs of the invention, recombinant antibodies of the invention, antibody-like proteins of the invention containing antibodies or active parts of antibodies, e.g. complementarity-determining regions, and chimeric proteins of the invention containing antibodies or active parts of antibodies and in addition heterologous proteins or parts thereof, that bind to the epitopes of the invention and that were therefore obtained, using the epitopes of the invention, can be identified by the skilled in the art, in similar experiments as described herein. In addition, the skilled in the art can identify epitopes of the invention, e.g. in a pharmaceutical composition for the immunisation of patients, using similar experiments as described herein.

The specificity of binding and the strength of binding of an antibody to the epitopes of the invention can be measured by any standard method known in the prior art, e.g. as described in K.O. Lloyd and V. Kudryasov, Tumor Biol 1998, 19 (suppl 1), 118-121 or by the kinetic measurement of antibody binding using the BIAcore 2000 apparatus (Pharmacia, Freiburg, FRG).

In the analyses of Table 1 and Table 2, an ELISA assay was used, using as antigens a panel of peptides, glycopeptids, synthetic oligosaccarides and different MUC1 fractions from normal, physiological sources. Antigens were used for the coating of EIA plates (Maxisorb, Nunc, Wiesbaden, FRG) at a concentration of 400ng/well. Wells were coated at room temperature for 18 hours. Wells were subsequently blocked with 1% BSA in PBS for 2 hours and then probed with Mabs diluted to 1000, 100, 10 or 1 ng/well. After probing for 1 hour at 37 °C, plates were washed and incubated with goat anti mouse IgG-peroxidase conjugates (1:20,000, Dianova, Hamburg, FRG). After washing, BMblue (Roche, BASEL,CH) was added as a substrate for 15 min and absorbance at 450 nm was measured.

**Table 1.**

| **Specificities of Mabs 7F11 and 1E4 and several well-known Mabs of the prior art for the epitopes of the invention** | | | | | | | | | | | |
|---|---|---|---|---|---|---|---|---|---|---|---|
| **ANTIGEN** | **αGalNAc** | **MONOCLONAL ANTIBODIES** | | | | | | | | | |
| | | **7F11** | **1E4** | BW 835 | MA 552 | 5D4 | VU 4H5 | BM2 | DF3 | HM FG1 | SM 3 |
| p1-10 | - | 0 | 0 | 0 | 0 | 0 | | ++ | | ++ | + |
| p1-10 | **T3** | **++** | **++** | 0 | + | 0 | 0 | ++ | | + | 0 |
| p1-10 | S9, T10 | 0 | 0 | 0 | | 0 | | + | | + | 0 |
| p11 -20 | T18, S19 | 0 | 0 | + | 0 | 0 | 0 | 0 | 0 | 0 | 0 |
| p17-7 | **T3** | 0 | **++** | 0 | | 0 | + | + | 0 | + | |
| (p17-14)x3 | - | 0 | 0 | 0 | + | 0 | + | ++ | ++ | ++ | + |
| p 1-24 | - | 0 | 0 | 0 | 0 | 0 | + | ++ | ++ | ++ | |
| p20-4 | - | 0 | 0 | 0 | 0 | 0 | 0 | + | + | + | + |
| αGalNAc | | 0 | 0 | 0 | 0 | + | 0 | 0 | 0 | 0 | 0 |
| BMA | | ++ | ++ | ++ | ++ | ++ | (+) | ++ | ++ | ++ | ++ |
| | | | | | | | | | | | |
| milk | | 0 | 0 | + | + | 0 | + | + | + | + | 0 |
| normal se | | | | + | | | | + | + | | |

Table 1 indicates the results of an analysis of the specificities of binding of Mab 7F11, Mab 1E4 and of several well-known Mabs of the prior art to the epitopes of the invention and to several control antigens. In Table 1, the binding specificities that were measured in the above-described ELISA assay as the absorbance at 450 nm, are indicated as:
- "0" no binding, corresponding to: A₄₅₀ < 0.25;
- "+" weak binding, corresponding to: 0.25 < A₄₅₀ < 1.0; and
- "++" strong binding, corresponding to: A₄₅₀ > 1.0.

In Table 1:
- the amino acid sequences of synthetic peptide antigens are indicated with reference to the MUC1 VNTR tandem repeat sequence indicated in Figure 1, e.g. "(p17-14)x3" indicates a polypeptide comprising three tandem repeats of a sequence comprising amino acids 17 to 20 and 1 to 14 in amino to carboxy-terminal direction.
- "αGalNAc" indicates the use of the disaccharide N-Acetyl-Galactosamine as antigen.
- "BMA" indicates the use of "breast mucin antigen" as antigen. BMA corresponds to tumor-associated underglycosylated MUC1 antigen produced the T47-D mammary carcinoma cell line, that was isolated by affinity chromatography, using Mab BM2 of the prior art with specificity for an unglycosylated peptide epitope of the MUC1 VNTR.
- "milk" indicates the use of physiological human breast milk from healthy individuals as antigen.
- "normal se" indicates a control, wherein physiological highly glycosylated mucin MUC1 from human urine was used as an antigen.
- glycosylation with a single αGalNAc residue in O-glycosidic linkage at any of the five potentially glycosylated serine (S) or threonine (T) residues of the MUC1 VNTR tandem repeat sequenee is indicated according to Figure 1 and using the One-Letter Code of amino acids, e.g. "T3" indicates threonine residue at position 3 of the MUC1 VNTR tandem repeat sequence carrying a single αGalNAc in O-glycosidic linkage.
- Mabs are indicated according to their names in prior art.

The data presented in Table 1 demonstrate, that:
- Mabs 7F11 and 1E4 are unique among the Mabs listed in Table 1, in that they bind strongly to a glycopeptide epitope of the invention comprising amino acids 1 to 10 of the MUC1 VNTR tandem repeat sequence indicated in Figure 1, wherein the threonine residue at position 3 carries a single αGalNAc residue in O-glycosidic linkage, but do not bind to an unglycosylated peptide comprising the same amino acids.
- Mab BM2 binds strongly to a peptide comprising amino acids 1 to 10 of the MUC1 VNTR tandem repeat sequence indicated in Figure 1, independently of the presence or absence of the αGalNAc moiety at the threonine residue at position 3, that is an essential structural feature of the epitopes of the invention. Mab BM2 does therefore not specifically bind to the tumor-associated epitopes of the invention.
- Mab HMFG1 binds strongly to a peptide comprising amino acids 1 to 10 of the MUC1 VNTR tandem repeat sequence indicated in Figure 1, only in the absence of the αGalNAc moiety at the threonine residue at position 3, that is an essential structural feature of the epitopes of the invention. Mab HMFG1 binds more weakly to the glycosylated epitopes of the invention than to unglycosylated peptides comprising the same amino acids as the epitopes of the invention.
- none of the Mabs listed in Table 1 binds strongly to a peptide comprising amino acids 1 to 10 of the MUC1 VNTR tandem repeat sequence indicated in Figure 1, wherein the serine residue at position 9 and the threonine residue at position 10 carry single αGalNAc residues.
- none of the Mabs listed in Table 1 binds strongly to a peptide comprising amino acids 11 to 20 of the MUC1 VNTR tandem repeat sequence indicated in Figure 1, wherein the threonine residue at position 18 and the serine residue at position 19 carry single αGalNAc residues.
- Mabs 1E4 binds strongly but Mab 7F11 does not bind to a peptide comprising amino acids 17 to 7 of the MUC1 VNTR tandem repeat sequence indicated in Figure 1, wherein the threonine residue at position 3 of the MUC1 VNTR tandem repeat sequence carries a single αGalNAc residue. As already mentioned above, this finding again demonstrates, that the epitope of Mab 1E4 is distinct from the epitope of Mab 7F11, in that the glycine residue at position 8 of the MUC1 VNTR tandem repeat sequence is not necessary for the binding of Mab 1E4.
- Mabs 7F11 and 1E4 do not bind to unglycosylated peptides of any length of the MUC1 VNTR tandem repeat sequence indicated in Figure 1.
- Mabs BM2, DF3 and HMFG1 bind strongly to unglycosylated peptide epitopes comprising at least amino acids 1 to 10 of the MUC1 tandem repeat sequence.
- αGalNAc is not recognised as such by any of the Mabs listed in Table 1, indicating, that Mabs 7F11 and 1E4 recognize the epitopes of the invention that contain the peptide structure of the epitopes of the invention in addition to the αGalNAc moiety.
- only Mabs 7F11, 1E4, 5D4 and SM3 possess high binding specificity for tumor-associated MUC1 antigen, i.e. they strongly bind to tumor-associated MUC1 antigen BMA and do not bind to mucin from healthy individuals.
- Mabs BW835, MA552, VU4H5, BM2, DF3 and HMFG1 possess low or no binding specificity for tumor-associated MUC1 antigen BMA.
- Mabs SM3 and 5D4 bind with high specificity to tumor-associated MUC1 antigen produced by mammary carcinoma cell line T47D as compared to mucin from healthy individuals, but bind only weakly to the unglycosylated peptides comprising parts of Sequence No. 1 indicated in Table 1, and do not bind to any of the glycosylated peptides of the MUC1 VNTR tandem repeat sequence, indicated in Table 1, including the epitopes of the invention.
- Mabs 7F11 and 1E4 are unique among the Mabs listed in Table 1, in that they do not bind to an unglycosylated (cryptic) peptide epitope of tumor-associated MUC1 antigen, but that their binding to the MUC1 VNTR region is strictly dependent on the presence of a single αGalNAc residue at one of the five potential glycosylation sites. The aforementioned αGalNAc residue must be present at the threonine residue at position 3 of the tandem repeat sequence of the MUC1 VNTR. Glycosylation of any of the four other potential glycosylation sites of the 20-amino acid tandem repeat sequence does not lead to the binding of Mabs 7F11 and 1E4.

**Table 2.**

| **Binding Affinities of Mabs 7F11 and 1E4 and several well-known Mabs of the prior art for the epitopes of the invention** | | | | | | | |
|---|---|---|---|---|---|---|---|
| **Mab** | **TYPE** | **ANTIGEN** | | | | | |
| | | BMA T47D | p1-10 | **p1-10 αGalNA c T3** | p1-10 αGalNAc S9+T10 | p11-20 αGalNAc T18+S19 | human milk |
| | | **Ka VALUE** | | | | | |
| **7F11** | IgG1 | 2.3E-8 | 0 | **1.5E-8** | 0 | 0 | 0 |
| **1E4** | IgG1 | 7.2E-8 | 0 | **2.4E-8** | 0 | 0 | 0 |
| 115D8 | IgG2b | 1.6E-9 | 0 | 0 | 0 | 0 | 2.2E-9 |
| BW835 | IgG1 | 6.7E-8 | 0 | 0 | | 2.3E-8 | 4.2E-8 |
| BM2 | IgG3 | 3.1E-9 | 1.2E-09 | 1.8E-9 | 1.5E-9 | | 3.4E-9 |
| DF3 | IgG1 | 4.5E-9 | nd | nd | nd | nd | 1.6E-9 |
| HMFG1 | IgG1 | 3.8E-9 | | 8.3E-8 | 2.0E-9 | | 4.1E-9 |
| SM3 | IgG1 | 7.1E-8 | | 0 | | | 0 |

Table 2 indicates the results of an analysis of the affinities of the binding of Mab 7F11, Mab 1E4 and of several well-known Mabs of the prior art to the epitopes of the invention and to several control antigens. In the analysis, the Ka value was determined, which is the antibody concentration at which the half maximal binding to an antigen is achieved. In the Table 2, the apparent K₅₀ values are indicted in the form of natural logarithms, e.g. "2.3E-8" meaning: 2.3 x e⁻⁸. In Table 2, the same abbreviations are used as in Table 1.

The binding affinities indicated in Table 2 demonstrate, that:
- all Mabs listed in Table 2 bind with high affinity to tumor-associated MUC1 antigen BMA produced by mammary carcinoma cell line T47-D, but only Mabs 7F11, 1E4 and SM3 do not bind to mucin from healthy patients.
- Mabs 115D8, BW835, BM2, DF3 and HMFG1 bind with comparably high affinity to tumor-associated MUC1 antigen produced by mammary carcinoma cell line T47D and to mucin from healthy patients, confirming the low specificity for tumor-associated MUC1 antigen of some of these Mabs already observed in the analysis of Table 1.
- Mab BW835 that does not possess specificity for tumor-associated MUC1 antigen, binds with high affinity to the glycopeptide comprising amino acids 11 to 20 of the MUC1 VNTR tandem repeat sequence that carries single αGalNAc residues on threonine 18 and serine 19, but does not bind to a peptide comprising amino acids 1 to 10 of the tandem repeat sequence of the MUC1 VNTR or to the epitopes of the invention.
- Mab BM2 that does not possess specificity for tumor-associated MUC1 antigen, binds with high affinity to a peptide comprising amino acids 1 to 10 of the MUC1 VNTR tandem repeat sequence, independently of the presence or absence of αGalNAc residues on threonine 3 or serine 9 and threonine 10.
- Mab SM3 that possesses high and specific affinity for tumor-associated MUC1 antigen, does not bind the glycopeptide epitopes of the invention, in particular it does not bind to the epitope of Sequence No. 3, used in the analysis of Table 2.
- Mabs 7F11 and 1E4 are unique among the Mabs listed in Table 2, in that they bind with high specificity and with high affinity to the glycopeptide epitopes of the invention.

The tumor specific glycopeptide epitopes of Mabs 7F11 and 1E4 of the invention possess unexpected features over the prior art. In the prior art it is expected, that the threonine residue at position 3 of the MUC1 VNTR tandem repeat sequence indicated in Figure 1 is unglycosylated in tumor-associated MUC1 antigen, because only peptide epitopes have been detected in the hydrophilic PDTRPAP region, comprising positions 1-7 (Pro-Asp-Thr-Arg-Pro-Ala-Pro) of the MUC1 VNTR tandem repeat sequence shown in Figure 1:
- A) In one study, 15 tumor specific Mabs which cross-react with the 20-amino acid tandem repeat domain of MUC1 were shown to cross-react with overlapping unglycosylated peptidepitopes within an immunogenic sequence PDTRP (J. Taylor-Papadimitrijou, Int. J. Cancer 1991, 49, 1-5)- comprising amino acids 1 to 5 of the immunodominant hydrophilic PDTRPAP region indicated in Figure 3;
- 14 Mabs cross-reacted with the unglycosylated sequence PDTRP, one Mab cross-reacted with the C-terminally overlapping epitope RPAP;
- the author concluded that the thereonine residue within the sequence PDTRP is not glycosylated in tumor-associated MUC1 antigen.
- B) In another study it was shown, that most Mabs made to breast cancer, which react with the protein core of MUC1, react with the synthetic unglycosylated peptide APDTR (V. Apostopoulos and F.C. McKenzie, Crit. Rew. Immunol. 1994, 14(3&4), 293-309) - comprising residues 20 and 1 to 4 of the VNTR tandem repeat sequence shown in Figure 1.
- C) In another study 56 Mabs were investigated, 34 of which cross-reacted with unglycosylated synthetic peptides located within the 20-amino acid tandem repeat sequence of the MUC1 mucin protein core (Price et al, Tumor Biol 1998, 19 suppl 1, 1-20, table 5);
- 30 of the aforementioned Mabs cross-reacted with epitopes of unglycosylated synthetic peptides, which partially overlapped the sequence PDTRP (residues 1 to 5 of the sequence in Figure 1) and contained the sequence TR (residues 3 and 4 of the sequence in Figure 1).

In addtition, in the prior art many carbohydrate specific as well as peptide specific Mabs are supposed to bind to conformational epitopes only expressed in multimeric tandem repeat structures (overview by Hilgers 1998 and Finn, patent US 5,827,666). Here we show that surprisingly a very short MUC1 peptide fragment with the sequence PDT(-O-αGalNAc)RPAPG (Sequence No. 2) that is substituted with only a single carbohydrate residue, namely α-N-acetylgalactosamine(αGalNAc) at the threonine residue in the aforementioned sequence, forms a tightly tumor-associated epitope of Mabs 7F11 and 1E4.

The above-mentioned studies of the prior art demonstrated that the threonine residue of the immunodominant PDTRP sequence of the 20-amino acid tandem repeat sequence of MUC1 is recognised in its unglycosylated form by a significant number of Mabs generated with MUC1 antigen from different tumor cell lines, secreted and partially purified antigen isolated from serum, ascites or effusions of tumor patients and with different synthetic MUC1 VNTR peptides. These findings strongly indicated that the threonine residue within the PDTR sequence is unglycosylated in tumor-associated MUC1. The epitope specifically bound by Mabs 7F11 and 1E4 on aberrantly tumor-associated underglycosylated MUC1 antigen, as disclosed herein, was therefore completely unexpected in the above-cited prior art.

The invention devises the structures of the carbohydrate-containing epitopes of tumor-associated underglycosylated MUC1 antigen, which are specifically bound by Mabs 7F11 and 1E4.

The epitopes of the invention enable the easy, fast and reliable production of further Mabs of the invention with the binding specificity of Mabs 7F11 and 1E4, in particular but without limitation those obtained in the classical mouse, rat and human hybridoma technique.

Preferred Mabs, immunologically active proteins and cellular receptors of the invention, e.g. T cell receptors, specifically cross-react with the tumor-associated MUC1 epitopes of the invention, in that they bind more strongly to tumor-associated MUC1 antigen than to normal physiological MUC1 antigen from healthy individuals. In particular, Mabs or T cell receptors of the invention specifically cross-reacts with tumor-associated MUC1 antigen, do not cross-react with unglycosylated peptide fragments of MUC1 of any length (4mer,11-mer, 24-mer, 60-mer and 105-mer synthetic peptides of the MUC1 VNTR), and do not cross-react with physiological fully glycosylated MUC1 antigen, such as MUC1 from human breast milk. Preferred Mabs, antibodies, proteins carrying parts of antibody molecules or T cell receptors of T cells of the invention that are obtained, using epitopes of the invention, bind to tumor-associated underglycosylated MUC1 antigen with comparable specificity as Mabs 7F11 and 1E4.

The monoclonal antibodies of the invention are preferably - but without any limitation to the invention - produced, as described in E. Harlow and D. Lane, Antibodies: A Laboratory Manual, Cold Spring Harbor Press (1988); Bessler et al., Immunobiol. 170 (1985) 239-244; Jung et al., Angewandte Chemie 97 (1985) 883 or Cianfriglia et al., Hybridoma Vol. 2(1993) 451-457. In a preferred method of the invention for the production of further Mabs like Mab 7F11 and/or Mab 1E4, experimental animals are in a first step immunised with an antigen of the invention, comprising an epitope of the invention of tumor-associated MUC1 antigen. In a second step, antisera from immunised experimental animals are obtained, or spleenocytes are isolated for the generation of hybridomas, from which in a later step monoclonal antibodies are obtained, wherein the epitopes of the invention are again used.

In a preferred method for the production of the Mabs of the invention, KLH-glycopeptide antigens of the invention are used for the immunisation of experimental animals. The KLH-glycopeptide antigens of the invention comprise the epitopes of the invention according to Sequences No. 1, 2, 3, 4, 5 and 6 that are covalently attached to keyhole limpet hemocyanin (KLH, Biosyn, Fellbach, FRG). In a first step of a preferred method for the production of the KLH-glycopeptide antigens of the invention, the epitopes of the invention according to Sequences No. 1, 2, 3, 4, 5 are synthesised - without any limitation to the invention - e.g. using the solid phase methods of the prior art. In these methods, threonine at position 3 in Sequences No. 1, 2, 3 and 6 or at the corresponding positions 4 in Sequence No. 4 and 5 in Sequence No. 5 is introduced e.g. as (N-f-Fmoc-O-β-(2acetamido-2-deoxy-3,4,6,trio-acetyl-f-D-galactopyranosyl)-L-threonine (Bachem, Heidelberg, FRG). The glycopeptides are purified if necessary, e.g. by HPLC. In a further step, the glycopeptide epitopes of the invention are covalently attached to keyhole limpet hemocyanin (KLH, Biosyn, Fellbach, FRG) or - for the applications described below - to bovine and human serum albumin (BSA and HSA), using e.g. bifunctional m-maleiimido-benzoyl-N-hydroxysuccinimid ester (Sigma, Deisenhofen, FRG) according to established methods (Kitagawa, 1981).

The KLH-glycopeptide antigens of the invention are readily used by the skilled in the art for the immunisation of experimental animals. In a preferred embodiment, mice are immunised with the above-described covalent KLH-glycopeptide antigen conjugates comprising any of the glycopeptide epitopes of Sequence No. 1 to 6 and KLH. Spleenocytes are fused 3-4 days after immunisation, e.g. as described in described in WO 99/40881, with mouse (X63Ag853 cells) or other myeloma cells. Hybridomas are selected in HAT-Medium. Hybridomas are screened for the production of Mabs of the invention that bind to the epitopes of the invention. Supernatant are analysed for MUC1 specificity after 10 to 14 days by ELISA using BSA- glycopeptide conjugate antigens of the invention and normal milk MUC1 as test antigens.

In preferred screening methods of the invention, the glycopeptide epitopes according to any of Sequences No. 1 to 6 are covalently linked to Bovine Serum Albumine (BSA) and used in the screening, selection or identification of hybridomas or any other cellular clone producing proteins that specifically bind to the epitopes of the invention. In a preferred embodiment, the epitopes of the invention of any of Sequences No. 1 to 6 comprise an additional cysteine residue at their C-terminal ends that is reactive with a bi-functional maleimide linker, that is reactive with BSA. The resulting BSA-glycopeptide antigens of the invention are used in a preferred method of the invention for the screening of the above-mentioned hybridomas and other cellular clones, producing proteins that specifically bind to the epitopes of the invention.

In a preferred method for the screening, selection or identification of said hybridomas or cellular clones, the BSA-glycopeptide antigens of the invention are used in ELISA assays of the invention, and in particular are used for the coating of ELISA plates ELISA assays of the invention are easily carried through in a variety of modifications by the skilled in the art, once that the epitopes of the invention have been provided.

The invention comprises, but is not limited to, the following antibodies, that specifically cross-react with the epitopes of the invention according to Sequences No. 1 to 6: antisera obtained from the blood of experimental animals; antibody molecules of any degree of biochemical purification, such as monoclonal antibodies (Mab); fragments of antibody molecules, such as Fab', F(ab)₂', obtained e.g. by enzymatic cleavage; any part or parts of antibody molecules, carrying at least the complementarity-determining regions (CDR) of the variable regions of the heavy or light chains of an antibody molecule, or carrying at least the CDR3 region of the variable region of the heavy or light chain of an antibody molecule, that can be obtained by any biochemical method or protein chemical procedure for the alteration of the structure of antibody molecules.

The antibodies of the invention comprise entire monoclonal antibodies, fragments thereof (e.g. Fv, (Fv)₂, Fab, Fab', F(ab)₂), chimeric, humanised or human antibodies as long as they are binding to MUC1 in a suitable manner. Short-chain antibody fragments containing only the CDR regions or parts thereof conferring the specific binding to MUC1 are preferred, if the antibody is a labelled one. Antibodies of the IgG1 isotype are preferred.

Antibodies of the invention comprise in addition any antibodies, proteins or polypeptides comprising at least one complementarity-determining (CDR) region of an antibody of the invention, that specifically binds to an epitope of the invention, that are produced by cell clones contained in and/or selected from antibody expression libraries. Said expression libraries comprise multitudes of bacterial, archeal or eucaryotic cells that have been genetically altered, e.g. by transformation, transfection etc., and contain recombinant DNA constructs comprising an expression vector and a gene or gene fragment that encodes an antibody, protein or immunologically active polypeptide comprising at least one complementarity-determining (CDR) region of an antibody.

In a further embodiment of the invention, the epitopes of the invention enable the easy, fast and reliable production of further Mabs of the invention and of recombinant proteins of the invention with similar binding specificities as Mabs 7F11 and 1E4, by the immunisation of transgenic mice expressing the human lg repertoire (Medarex, USA) with the epitopes of the invention, and by the screening of phage display libraries (Griffiths and Hoogenboom, 1996) producing human antibodies and antibody fragments (as described in patent of Cambridge Antibody Technology, Melbourn, UK) with the epitopes of the invention. The epitopes of the invention enable in addition the easy, fast and reliable production of fully synthetic antibodies, wherein human combinatorial antibody libraries (HuCAL technology, Morphosys, München FRG) are screened with the epitopes of the invention. All the aforementioned immunologically active proteins can be easily produced, once that the epitopes, that are specifically bound by Mabs 7F11 and 1E4, have been disclosed in the invention.

In the prior art, libraries have been devised which contain enormous numbers of genetically engineered clones of vector/host systems expressing recombinant genes for individual Mabs or antibody fragments with different specificities (up to 10¹⁰). Such libraries have been described, by displaying antibody light and heavy chains as scFv-fragments at the surface of filamentous phages ( Breitling 1991 and Hoogenboom 1991).

With these libraries there exists the problem of selecting the desired clonotypes. The enormous number of clones that form part of such libraries make sure, that Mabs that bind to most naturally occurring antigens may well be produced by some clone of such a library. However, the cross-reactivities of Mabs of individual clones are not known and the frequency of specific clonotypes is very low. In order to identify and isolate individual clones that produce Mabs or fragments with MUC1 glycopeptide specificity it is necessary to execute highly sophisticated screening/panning methods for the selection of the individual clones which are necessarily present in very small concentration within the enormous number of different clones contained in such a library.

Therefore, the step of selection within an above-mentioned library may be very time-consuming and in theory offers no advantage as compared to the selection of a classical hybridoma producing a desired Mab, as described above. In fact, abs or fragments of abs which retain at least part of the specific cross-reactivity of a Mab with a defined specificity may be even more difficult to select from an above-mentioned libraries than from a mixture of classical hybridomas, given the lower concentration and lower stability of abs or fragments which retain at least part of the specific cross-reactivity.

In a preferred embodiment of the invention methods are provided for the selection of clones from said expression libraries that produce Mabs or parts or fragments of Mabs which retain at least part of the specificity for the epitopes of the invention as Mabs 7F11 and/or 1E4. In a preferred method for the screening, selection or identification of cellular clones in the above-mentioned libraries, the BSA-glycopeptide antigens of the invention are used in ELISA assays of the invention, and in particular are used for the coating of ELISA plates. ELISA assays of the invention are easily carried through in a variety of modifications by the skilled in the art, once that the epitopes of the invention have been provided.

Panning techniques and repeated rounds of clonal selection have been successfully used for the isolation of recombinant antibodies (Winter, 1991, Marks, 1992). The glycopeptides of the invention can be produced with high precision in unlimited amounts and can be applied in all known selection systems of recombinant phage antibody systems (RPAS, Cambridge Antibody Technology LtD, Cambridge,UK and HuCAL technology, Morphosys, München FRG). Affinity selection with the glycopeptides of the invention is performed according to the invention, using biotinylated glycopeptide epitopes of the invention as antigen, magnetic separation or solid phase affinity techniques. In another preferred embodiment, the above-described ELISA assays of the invention can be applied, wherein conjugates of BSA and the epitopes of the invention are used.

In a preferred embodiment of the invention, the glycopeptide epitopes of the invention are used for immunisation of transgenic mice (or other species) of the prior art, expressing human immunoglobulin genes (Jacobovits, 1995, Lonberg, 1995). Different immunisation protocols can be used, wherein any of the glycopeptide epitopes, in particular the above-described covalent KLH conjugates of glycopeptides of any of Sequences No. 1 to 6 are used, optionally with additional adjuvants. Specific antibodies of hybridomas are screened and selected with the above-mentioned methods wherein the glyocpeptides of Sequence No. 1 to 6 are conjugated with BSA, biotin, magnetic beads or solid substrates.

The invention comprises in addition any of the aforementioned antibodies, monoclonal antibodies, fragments of antibody molecules, and parts of antibody molecules of the invention, that specifically cross-react with tumor-associated MUC1 antigen, that can be obtained by molecular biological methods, e.g. by the expression of suitably manipulated and rearranged DNA molecules, such as, e.g. cDNAs, genes, gene fragments, and exons, that encode the above-mentioned antibodies, monoclonal antibodies, fragments of antibody molecules, part or parts of antibody molecules, i.e. CDR-grafting, that have been integrated into suitably chosen expression vectors, in suitably chosen bacterial, archeal or eucaryotic host cells. Recombinant proteins of the invention and recombinantly produced antibodies of the invention may be prepared on the basis of the sequence data according to methods known in the art and described in Sambrook et al., Molecular Cloning: A Laboratory Manual, 2nd edition (1989) Cold Spring Harbor, New York, and Berger and Kimmel, Methods in Enzymology, Vol. 152, Guide to Molecular Cloning Techniques (1987), Academic Press Inc., San Diego, CA.

Monoclonal antibodies of the invention and recombinant proteins of the invention that bind specifically to the epitopes of the invention are in addition obtained by the expression in suitable vector/host systems of the genes, gene fragments etc., encoding antibodies or parts or fragments of antibodies specifically cross-reacting with tumor-associated MUC 1 antigen, that are manipulated, rearranged or recombined by molecular biological methods with additional genes or gene fragments in any desired way. In this case, expression of the recombined genes in suitable vector/host systems leads to the production of antibody molecules with structural alterations, e.g. the so-called "single-chain fragment variable antibodies" (scFv) of the prior art. (overview Bird, 1988, Houston 1988).

Preferred embodiments of the invention are fragments of Mabs, antibody-like proteins and chimeric proteins, obtained by the expression of recombinant genes encoding parts of Mabs that cross-react with the epitopes of the invention, in suitable vector-host systems. The isolation, manipulation, including predetermined rearrangements, and expression of genes encoding the Mabs of the invention, involves only the use of routine methods for the skilled in the art, once that the epitopes of the invention have been disclosed that enable the selection of hybridomas producing the desired Mabs.

Recombinant proteins of the invention can be expressed in eukaryotic or prokaryotic host cells according to standard methods known in the art; preferably mammalian cells, such as lymphocyte cell lines, may be used as host cells. Typically, recombinant nucleic acid constructs encode a complete human antibody heavy chain or a complete human antibody light chain or a heavy or light chain variable region or at least one complementarity-determining region that mediates binding to the glycopeptide epitopes of the invention. Alternative human constant region sequences (heavy and/or light chain) other than those naturally associated with said antibody chains may be substituted, including human constant region isotypes, such alternative human constant region sequences can be selected e.g. from E.A. Kabat et al., Sequences of Proteins of Immunological Interest (1987), National Institute of Health, Bethesda, MD.

In preferred embodiments of the invention additional genes or gene fragments are recombined by the skilled in the art with genes or gene fragments encoding the above-mentioned antibodies or recombinant proteins of the invention, that bind to the epitopes of the invention. Said additional genes comprise e.g. heterologous genes or fragments of genes, cDNAs, exons etc., encoding different proteins, such as genes for any cellular membrane receptors, signal transducing membrane receptors, T cell receptor chains, costimulatory molecules of the T cell stimulation pathways, the Fc receptor, any protein toxin or bacterial superantigen, any enzyme or part of an enzyme, and any other desired protein or part, region or domain of a protein , including but not limited to viral, and bacterial proteins useful for tumor diagnosis or tumor therapy may be linked to an antibody or antibody-like protein or polypeptide, that specifically cross-reacts with tumor-associated MUC1 antigen. An example of such a recombinant protein is described by Sakurai (1999). The superantigen SEA, a potent T cell activator, was produced by gene engineering and fused to a scFv gene of Mabs MUSE11 with specificity for the MUC1-peptide. Recombinant reSEA-MUSE11scFc was produced in E. coli and found to react with MUC1 and with MHC class II antigens of T cells.

Preferred embodiments of the invention are in addition antibody-toxin conjugates comprising Mabs, antibody-like proteins or chimeric proteins of the invention that are covalently coupled to toxin molecules, e.g. such like Pseudomonas exotoxin A (Schmidt, 1998), bacterial superantigen SEA (Shinoda, 1998), antibody-ribonuclease conjugates ( ) and recombinant bi-specific antibodies or diabodies by combining anti-MUC1 with anti-CD3 antibodies or fragments (Zhang 1998).

In a preferred embodiment of the invention, an antibody of the invention or part of it is conjugated or translationally fused to a toxin molecule (immunotoxin), thus effecting specific killing of tumor cells (Brinkmann et al., Proc. Natl. Acad. Sci. USA 88 (1991) 8616-8620; Pastan et al., Cancer Res. 51 (1991) 3781-3787; FitzGerald and Pastan, J. Natl. Cancer 81 (1989) 1455-1461) or conjugated to a cytokine or interleukin. In another preferred embodiment of the invention, bi-specific antibodies are used for tumor therapy (Bonino et al., BFE 9 (1992) 719-723), which may be constructed by in vitro re-association of polypeptide chains, by hybrid hybridoma generation or by construction of diabodies (Holliger et al., Proc. Natl. Acad. Sci. USA 90 (1993) 6444-6448; Holliger and Winter, Current Opin. Biotechnol. (1993) 446-449).

With regard to immunotoxins, it is preferred in the invention to couple the antibody according to the invention to a toxin, such as, for example, Pseudomonas exotoxin, Diphtheria toxin or other toxins (FitzGerald and Pastan, J. Natl. Cancer 81 (1989) 1455-1461). It is also preferred to couple the antibodies to chemotherapeuticals, such as, for instance, doxorubicin, or to radioactively labelled substances which have a cytotoxic effect. Conjugates of the antibodies according to the invention, in particular of human antibodies, for in vivo imaging, using, for instance, radioactive or fluorescent substances, are also preferred.

In a preferred embodiment of the invention, an antibody or a fragment thereof (normally generated proteolytically, e.g. Fab-fragment) is chemically coupled in vitro to a toxin or toxin fragment. For practical reasons, the antibody part in this type of immunotoxin is either a complete antibody (consisting of two light and two heavy chains) or, more preferably, a Fab-fragment (consisting of one light chain and the VH- and CH1-regions of the heavy chain).

In a further preferred embodiment of the invention, immunotoxins are generated by recombinant DNA techniques, which favourably leads to a defined, homogeneous molecule. The size of the antibody part is preferably as small as possible to obtain a small immunotoxin with good tissue penetration. In this method, the smallest practically available antibody fragment is not the Fab-fragment, but the functional variable domain of an antibody, consisting of the VH-region of the heavy chain and the VL-region of the light chain only. VH-and VL-region (polypeptide chains each of about 100 amino acids) have to form a functional assembly, the variable domain, which confers antigen binding. In the absence of any of the remaining parts of an antibody, VH- and VL-region form very labile complexes only. Therefore, their complex is preferably stabilised by covalent bonds.

In a preferred embodiment, gene fragments encoding VH-region, VL-region (or vice versa) and a toxin moiety are fused by the skilled in the art and expressed in a suitable expression system. Upon expression, a single polypeptide chain is formed, wherein VH- and VL-region, being connected by a peptide linker, fold into a stable variable domain, while the toxin is fused e.g. to VL via a second peptide linker (see Brinkmann et al., Proc. Natl. Acad. Sci. USA 89 (1992) 3075-3079). The length of both peptide linkers is variable and may in some instances even be reduced to a single peptide bond. A molecule of this type has been termed a "single chain immunotoxin", analogous to the term "single chain antibody" or scFV, which is used for a single polypeptide chain containing both VH and VL connected by a peptide linker or bond.

The invention comprises, of course, additional possibilities to produce immunotoxins by recombinant DNA techniques, for instance by using the larger Fab-fragment (VH-CH1 non-covalently assembled to VL-CL, while one of them is fused by a peptide linker to the toxin). However, the possibilities described by Brinkmann et al., Proc. Natl. Acad. Sci. USA 89 (1992) 3075-3079 and Brinkmann et al., Proc. Natl. Acad. Sci. USA 90 (1993) 7538-7542 are to be preferred. With respect to the toxin part of the immunotoxin, preferred fragments of the Pseudomonas exotoxin (PE) are PE38 and PE40 and derivatives thereof (I. Pastan et al., WO 92/07271, WO 90/12592).

The present invention provides in addition the epitopes of the invention as valuable tools for the selection and generation of B-cells of the invention and dendritic cells of the invention and NK-cells of the invention, as well as for in vitro and in vivo selection and generation of effector NK and T cell clones of the invention, that cross-react with similar specificity with tumor-associated underglycosylated MUC1 antigen as the aforementioned Mabs 7F11 and 1E4, and that can be easily generated with the methods of the prior art, once that the epitopes that are specifically bound by Mabs 7F11 and 1E4, have been disclosed in the invention.

In preferred embodiments of the invention, effector cells involved in the cellular immune response of the body are generated, that are cross-reactive with the epitopes of the invention, either directly or in antigen presentation, and are used prophylactically or therapeutically against MUC1 positive tumours. T cell receptors of T cells specifically cross-react with or bind to tumor-associated MUC1 antigen by binding to a complex comprising a fragment of tumor-associated MUC1 antigen, bound by and presented to it by a Major Histocompatiblility Complex (MHC) molecule of an antigen-presenting cell (APC like macrophages, dendritic cells, B-cells etc.). T cells carrying T cell receptors, specifically cross-reacting with tumor-associated MUC1 antigen can e.g. be obtained by ex vivo-stimulation and clonal in vitro-expansion using T cells from peripheral blood mononuclear cells (PBMC) or bone marrow of normal and tumor patients.

In a preferred method for the production of the effector T cells and NK cells of the invention, the above-described KLH-glycopeptide antigens of the invention are used for the ex vivo stimulation and in vitro selection of T cells or NK cells of an individual. The skilled in the art that is provided the epitopes and/or KLH-glycopeptide antigens of the invention can readily generate the syngeneic tumor specific effector T cells and NK cells of the invention. In the case of effector T cells, growth factors, e.g. IL-2, and/or cytokines influencing the type of immune response, e.g. IL-4 or IFN-γ are added to the culture medium.

In preferred embodiments of the invention the epitopes of the invention are used for the specific ex-vivo-stimulation and/or selection of syngeneic T cells of an individual and for the generation of highly tumor specific syngeneic effector T cells of the invention. Said syngeneic tumor specific effector T cells of the invention are obtained in about one to two weeks, by stimulation of T cells of an individual in the presence of any of the epitopes of the invention, and if necessary in the presence of antigen-presenting cells, suitable stimulatory cytokines, such as e.g. IL-2. The methods for the ex vivo stimulation of T cells of the invention allow for the first time the generation and/or selection of syngeneic effector T cells, in particular
a) CD4 positive MHC-2 positive T helper cells
b) inflammatory CD4 positive MHC-2 positive T cells
c) cytotoxic CD8 positive MHC-1 restricted T cells for individual patients, that possess the specificity of monoclonal antibodies for tumor-associated MUC1 antigen. In addition the invention comprises the addition of cytokines, such as IL-12, IFN-γ or IL-4 to the in vitro culture of T cells, that induce a desired phenotype in the effector T cells, namely the induction of the known th1 or th2 phenotypes, respectively.

In further preferred-embodiments of the invention, genetically altered cells of the invention of the immune system, carrying T cell receptors of the invention that specifically cross-react with the tumor-associated MUC1 epitopes of the invention, are generated by the expression of TCR α and/or β genes of the invention, that are isolated from T cells, that are cloned using the epitopes of the invention, in a suitable vector-host system comprising a predetermined expression vector and a suitable host cell type. The invention comprises the expression of the TCR α and/or β genes of the invention in any desired human or animal cell of the immune system, involved in humoral or cellular immunity.

In a preferred embodiment of the invention, fragments of the aforementioned TCR α and β genes of the invention are by molecular biological methods manipulated, rearranged and ligated in any desired way to the genes encoding any other human or animal transmembrane proteins, which are anchored to the cell membrane. By the expression of the aforementioned rearranged chimeric genes of the invention in suitable vector/host systems, the ability of T cell receptors to specifically cross-react with tumor-associated MUC1 epitopes of the invention, is conferred to other cellular receptor proteins. The invention comprises the expression of the aforementioned chimeric membrane receptor protein genes of the invention in any desired human or animal cell of the immune system, involved in humoral or cellular immunity.

In further preferred embodiments of the invention, the Mabs and the other above-described immunologically active proteins of the invention are used in the diagnosis of tumours which produce underglycosylated MUC1. The Mabs of the invention are used in particular in the methods of the prior art, e.g. Enzyme-Linked Immuno-Sorbent Assays (ELISA), Western blot analyses, BiaCore and any other immunological detection method, involving at least one step of specific cross-reaction of an antigen-antibody pair, consisting of tumor-associated underglycosylated MUC1 as antigen and an antibody of the invention.

The antibodies of the invention are used in diagnostic methods comprising an immunological determination method, such as are well-known in the prior art and can be readily used or can be readily devised by the skilled in the art, once that the antibodies of the invention are provided. In particular, the antibodies of the invention are used in diagnostic methods as unlabelled and/or immobilised receptors.

Immobilised Mabs, antibody fragments or antibody-like proteins of the invention are preferably used in a method for the removal of tumor cells or secreted MUC1 antigen from a patient's body material, wherein the body material is brought into contact with an immobilised antibody of the invention, the tumor cells or said secreted MUC1 antigen bind to the immobilised antibody, and the tumor cells or said secreted MUC1 antigen bound to the solid phase are separated along with the solid phase from the body material.

In a preferred embodiment of the invention, the Mabs and recombinant proteins of the invention are used for the measurement of secreted tumor-associated MUC1 antigen in the circulation. Their measurement in blood provides a guide to tumor burden and repeated determinations reflect disease recurrence, progression or response to therapy.

The Mabs and other immunologically active agents of the invention enable the detection of so-called "micrometastases" in tissue samples from tumor patients that had underwent surgical treatment and/or chemotherapy and that still carry very small residual aggregates of tumor cells, that have survived chemotherapy and that might with a high risk be resistant to the pharmaceutical agent used in chemotherapy

In a preferred embodiment of the invention, the epitopes and BSA-glycopeptide antigens of the invention are used for the determination of the presence and/or of the concentration of Mabs that specifically bind to the epitopes of the invention in the circulation or a tissue sample of a patient or a healthy person. A preferred method for said determination comprises the use of the above-described BSA conjugates of the glycopeptide epitopes of the invention, e.g. in ELISA or BiaCore assays or any other antibody-detection assay, that the skilled in the art devises, that has been provided the epitopes of the invention.

Preferably, the above-mentioned determination methods are used to determine the presence of endogenous antibodies or effector T cells, NK cells etc. that specifically bind to the epitopes of the invention, in the circulation of a patient. In a further preferred embodiment, the above-mentioned determination methods are used to determine the presence and/or concentration of antibodies against the epitopes of the invention in the circulation of an individual that has received the Mabs, antibodies, recombinant or chimeric proteins of the invention in passive immunisation or that has received the glycopeptide epitopes or vaccines of the invention in active immunisation, as described below.

In a particularly preferred embodiment of the invention, the BSA-glycopeptide antigens of the invention are used in a diagnostic test. In the diagnostic test of the invention, the presence of antibodies against the epitopes of the invention is determined with the methods disclosed herein. In a preferred diagnostic test of the invention, the BSA-glycopeptide antigens of the invention comprising any of the epitopes of Sequence No. 1 to 6, in particular comprising the epitopes according to Sequence No. 4, 5 or 6, are used in an ELISA assay.

A preferred embodiment of a diagnostic test of the invention comprises a first step, wherein a blood or tissue sample is removed from the body of a patient and the presence of a tumor that produces aberrantly glycosylated MUC1 antigen is determined, using Mabs 7F11, 1E4 and/or the antibodies of the invention. If a tumor that produces aberrantly glycosylated MUC1 antigen has been detected, the presence of antibodies against the epitopes of the invention in a blood or tissue sample of said patient is determined. Said second step comprises the above-described use of an epitope of the invention or of a BSA-glycopeptide antigen of the invention. The aforementioned diagnostic tests of the invention are of prognostic value in the diagnosis of MUC1-producing tumours, because they allow to determine, whether an effective immune response against the tumor-associated MUC1 antigen epitopes of the invention has occurred in said patient. The diagnostic tests of the invention are therefore of enormous prognostic value and help to determine appropriate therapeutical approaches.

In further preferred embodiments of the invention, the Mabs, the other above-described immunologically active proteins or cells of the invention, e.g. effector T cells, NK cells, the glycopetide epitopes, tumor vaccines and pharmaceutical compositions of the invention are used in the prophylactical immunisation and therapy of tumours which produce underglycosylated MUC1.

The Mabs of the invention are used for the treatment of micrometastases in tumor patients in so-called "adjuvant therapy", immediately or soon after surgical and chemotherapeutical elimination of the so-called "primary tumor", in order to eliminate residual resting and/or resistant tumor cells, using immunological methods of the invention to a) specifically detect the micrometastatic tumor cells and b) either directly inactivate and destroy the detected cells, e.g. by the activation of the physiological complement system by using a therapeutic Mab of the invention of the IgG isotype, carrying a heavy chain with a constant part (Fc) that is able to activate the complement system, or by using an immunotoxin of the invention (see below), or c) mediate killing of the detected micrometastatic cells by the effector cells of the cellular immune system (see below).

In a preferred embodiment the Mabs, glycopeptide epitopes and tumor vaccines of the invention are used for the in-vivo-immunisation and for the elicitation of humoral and/or cellular immune responses against tumor-associated MUC1 antigen. Suitable immunogens are e.g. the above-described covalent conjugates of KLH and the epitopes of the invention.

In a preferred embodiment, the Mabs of the invention are used for the passive immunisation of patients according to established methods, e.g. as described in WO 99/40881. Passive immunisation of patients has the advantage to be independent of the patient's own immune response and thus, antibodies are immediately available. Passive immunisation can in addition be applied in patients with a compromised immune system. Given the defined epitope of the invention, the antiserum advantageously possesses a comparable specificity for tumor-associated MUC1 antigen as monoclonal antibodies.

The Mabs of the invention are used in passive immunisation, using murine, humanised or human monoclonal antibodies (Mabs) of the invention, that cross-react with the defined tumor-associated MUC1 antigen epitopes of the invention and are formulated into pharmaceutically acceptable injection or infusion solutions. According to preferred embodiments of the invention human monoclonal antibodies of the invention are either selected from expression libraries of human antibodies, using the epitopes of the invention, or are obtained by a procedure for the "humanisation" of the Mabs of the invention, that specifically bind to the epitopes of the invention. The skilled in the art can readily carry through the aforementioned selection methods of the invention and the humanisation procedures of the invention, once that the epitopes of the invention have been disclosed.

In another preferred embodiment of the invention, the carbohydrate-containing epitopes of the invention of tumor-associated MUC1 antigen are used for the active immunisation of patients, in order to generate a humoral and/or cellular immune response of a patient against tumor cells producing the cross-reactive MUC1 antigen comprising an epitope of the invention. In another preferred embodiment of the invention, healthy individuals are prophylactically immunised with the methods for active immunisation of the invention. In the prophylactical immunisations of the invention, an effective immune response is established in the body of said healthy individuals against tumours that produce aberrantly MUC1 comprising the epitopes of the invention.

In a preferred embodiment of the invention, healthy individuals, in particular those at high risk of developing breast, ovarian and endometrium carcinoma, are prophylactically immunised with a suitable antigen carrying any of the epitopes of the invention. In a second preferred embodiment of the invention, tumor patients are immunised, thereby enhancing their immune response against the MUC1-positive tumor cells in primary tumours, local metastases, micrometastases and generalised metastasis.

In further preferred methods of the invention for active immunisation, glycopepeptids according to any of Sequences No. 1 to 6 are used as tumor vaccine according to well known techniques. In particular glycopepeptids of the invention are used comprising multimers of the epitopes of the invention, which are arranged in the form of tandem repeats, or comprising any longer or modified MUC1 VNTR sequence. Suitable vaccines comprise the glycopeptides of the invention that are mixed with BCG (Goydos, 1996), and/or that are covalently linked to KLH and QS21 (Gilewski, 1998), and/or that are covalently linked to any other immunogenic peptides of viral, bacterial, fungal or other foreign or human origin that can be used as carrier protein and preferably also as adjuvant in active immunisations. In preferred tumor vaccines of the invention, glycopeptide epitopes of the invention comprising any of Sequences No. 1 to No. 6, and in particular the longer epitopes of Sequence No. 4 to 6, are linked to KLH and used for the immunisation of patients or healthy individuals. Preferred tumor vaccines of the invention comprise in addition a pharmaceutical carrier and/or an adjuvant.

In a preferred tumor vaccine of the invention, the 11-mer glycopeptide epitope of the invention of Sequence No. 4, or more preferably, the 12-mer glycopeptide eptitope of the invention of Sequence No. 5 carries a single additional cysteine residue at its C-terminal end. Said additional C-terminal cysteine residue is reactive with the above-mentioned bi-functional maleimide linker molecule. Key hole Limpet Hemocyanine (KLH) that has been modified with the bi-functional maleimide linker molecule can therefore be covalently coupled to said reactive additional cysteine residue at the C-terminal end of the epitope of the invention of Sequence No. 4 or No. 5. Thus, a preferred tumor vaccine of the invention comprises the epitope of the invention of Sequence No. 4 and more preferably of Sequence No. 5, that is covalently coupled via an additional cysteine residue at its C-terminal end and a bi-functional maleimide linker molecule to KLH.

In preferred embodiments of the invention, the epitopes of the invention are used for the active immunisation of tumor patients or healthy individuals, using methods known in the literature and already described for MUC1 peptides (Finn, US patent 5,827,666) and MUC1 glycoconjugates (Livingstone, 1995). In a preferred embodiment the glycopeptide epitopes of any of Sequences No. 1 to 6 the invention are covalently coupled to KLH and the above-described KLH-glycopeptide antigens of the invention are prepared. Patients or healthy individuals are vaccinated, using e.g. the methods known to the skilled in the art, preferably by 4 to 6 repeated subcutaneous applications of said KLH-glycopeptide conjugate antigens of the invention at individual dosages of 50 to 200 µg, admixed with an adjuvant (preferably DetoxTM-B-Stable Emulsion, Ribi Immunochem Res. Inc., USA) at 0, 2, 4, 6, 10 and 12 weeks (Miles, 1996).

In further preferred embodiments of the invention, the glycopeptide epitopes of Sequence No. 1 to 6 of the invention are used for the generation of a vaccine of the invention comprising tumor-associated MUC1 antigen epitope-transduced dendritic cells (DCs). Said transduced DCs are generated ex vivo from CD34 enriched peripheral blood cells from breast cancer patients undergoing high-dose chemotherapy and PBSCT (peripheral blood stem cell therapy). (Brugger, 1997, overview in Kanz, 1996). In preferred embodiments, DCs are "pulsed with the glycopeptide epitopes of the invention or are genetically altered to produce and present the epitopes of the invention. Preferably, the epitopes of the invention that comprise up to 9 amino acid residues are used for the generation of the DC vaccines of the invention.

Since the Mabs of the invention bind to the surface of tumor cells, they can be used for in vivo treatment in humans. Thus, invention also provides pharmaceutical compositions which comprise one or more antibodies of the invention, optionally together with conventional pharmaceutical carriers, adjuvants, filling or additive materials. The administration of a medicament according to the present invention is useful for the treatment of micrometastases, especially in the treatment of breast cancer.

Preferred dosages of the antibodies of the invention in therapeutic treatments comprises about 2 µg to 20 mg, preferably 50 µg to 20 mg /kg body weight. Preferrably said dosages are repeatedly administered.

For the treatment of patients with breast carcinoma with the antibodies according to the invention it is preferred to carry out, after the surgery, an established adjuvant chemotherapy (before menopause) or hormone therapy (after menopause) in lymph-node-positive patients (about 45%). In a further step, the presence of tumor cells in the bone marrow is diagnosed. This is done preferably with the use of the antibodies according to the invention, but can be accomplished also with other tests recognising usual tumor markers (cytokeratins, etc.). If the bone marrow reaction is positive, preferably 6 to 8 immunisations are performed at a 1 to 4-week-interval using the antibody according to the invention. The amount of antibody used is in the range of 10 to 200 mg per immunisation. This antibody therapy is carried out during adjuvant hormone therapy or after chemotherapy. In the case of hormone therapy, it may be carried out preferably in a parallel manner (no immunosuppression in the latter case).

Another determination of the tumor cells in the bone marrow is performed three months after the last immunisation. At least 6 x 10⁶ bone marrow cells are analysed for tumor cell contamination. It has been found that when using the antibodies according to the invention, 17 out of 18 patients exhibit negative reactions in the assay for tumor cells in bone marrow, which implies that the tumor cells in the bone marrow have obviously been destroyed as a result of the therapeutic application of the antibodies according to the invention. Surprisingly, the antibodies of the invention exhibit, in addition, an effector function, whereby, after binding to the tumor cells, the tumor cells are recognised as foreign cells and are presumably eliminated by the activation of the complement, anti-idiotypic antibodies, activated NK-cells or T-cells or a combination of the latter.

Therapeutic compounds of the invention may be administered parenterally, such as intravascularly, intraperitoneally, subcutaneously, intramuscularily, using forms known in the pharmaceutical art. The active drug components of the present invention are used in liquid, powdered or lyophilised form and may be combined with a suitable diluent or carrier, such as water, a saline, aqueous dextrose, aqueous buffer, and the like. Preservatives may also be added.

Regardless of the route of administration selected, the compounds of the present invention are formulated into pharmaceutically acceptable dosage forms by conventional methods known to those skilled in the art. The compounds may also be formulated using pharmacologically acceptable acid or base addition salts. Moreover, the compounds or their salts may be used in a suitable hydrated form.

Preferred embodiments of the invention are in particular:
1. Epitopes of tumor-associated MUC1 antigen comprising at least an amino acid sequence according to Sequence No. 1 and comprising at least the carbohydrate moiety at position 3 of Sequence No. 1.
2. Epitopes according to preferred embodiment 1, wherein the carbohydrate moiety at position 3 is a single αN-Acetyl-Galactosamine residue coupled in O-glycosidic linkage to the threonine or serine residue at position 3.
3. Epitopes according to preferred embodiments 1 or 2 comprising at least the amino acids and at least the carbohydrate moiety at position 3 of Sequence No. 2.
4. Epitopes according to any of preferred embodiments 1 to 3, which are specifically bound by monoclonal antibodies 7F11 and/or 1E4, which have been disclosed in the published International Patent Application WO 99/40881.
5. Epitopes according to preferred embodiment 4 comprising at least the amino acids and at least the carbohydrate moiety at position 3 of Sequence No. 3, and/or comprising at least the amino acids and at least the carbohydrate moiety at position 4 of Sequence No. 4, and/or comprising at least the amino acids and at least the carbohydrate moiety at position 5 of Sequence No. 5, and/or comprising at least the amino acids and at least the carbohydrate moiety at position 3 of Sequence No. 6.
6. Epitopes according to any of preferred embodiments 1 to 5 comprising at least one additional amino acid of a sequence comprising at least two copies of Sequence No. 6, which are arranged as tandem repeats.
7. A tumor vaccine comprising an epitope according to any of preferred embodiments 1 to 6, comprising an additional cysteine residue at its C-terminal end, that is linked by a bi-functional linker molecule to a carrier protein.
8. A tumor vaccine according to preferred embodiment 7, comprising an epitope according to Sequence No. 4, 5 or 6, a bi-functional maleimide linker and Key-hole Limpet Hemocyanine (KLH).
9. A pharmaceutical composition comprising a tumor vaccine according to preferred embodiment 7 or 8 and a pharmaceutical carrier and/or an adjuvant.
10. Use of a tumor vaccine according to preferred embodiment 7 or 8 and/or of a pharmaceutical composition according to preferred embodiment 9 for the immunisation of humans or animals.
11. Use of a tumor vaccine and/or a pharmaceutical composition according to preferred embodiment 10, wherein the human is a healthy individual or a tumor patient.
12. Use of an epitope according to any of preferred embodiments 1 to 6 and/or of a tumor vaccine according to preferred embodiments 7 or 8 and/or a pharmaceutical composition according to preferred embodiment 11 for the production of monoclonal antibodies.
13. A method for the production of monoclonal antibodies, wherein an epitope according to any of preferred embodiments 1 to 6 and/or a tumor vaccine according to preferred embodiment 7 or 8 and/or a pharmaceutical composition according to preferred embodiment 9, is used.
14. A method according to preferred embodiment 13, comprising at least one first step, wherein an experimental animal is immunised with an epitope according to any of preferred embodiments 1 to 6 and/or a tumor vaccine according to preferred embodiment 7 or 8 and/or a pharmaceutical composition according to preferred embodiment 9, and a second step, wherein hybridomas are selected using an epitope according to any of preferred embodiments 1 to 6 and/or a tumor vaccine according to preferred embodiment 7 or 8, in particular a modified tumor vaccine comprising bovine serum albumine as carrier protein.
15. A monoclonal antibody that differs from Mabs 7F11 and 1E4 in at least one amino acid, that is obtained with a method according to preferred embodiment 13 or 14.
16. A method for the production of a monoclonal antibody or a recombinant protein that binds to an epitope according to any of preferred embodiments 1 to 6, comprising at least one step, wherein a nucleic acid encoding said monoclonal antibody or recombinant protein is expressed in a vector-host system for the expression of genetic information.
17. A method according to preferred embodiment 16, comprising a first step, wherein a cellular clone that produces said monoclonal antibody is identified using an epitope according to any of preferred embodiments 1 to 6, a second step, wherein a nucleic acid encoding said monoclonal antibody is isolated from said cellular clone, a third step, wherein said nucleic acid is ligated to a nucleic acid vector molecule of a vector-host system for the expression of genetic information, a fourth step, wherein the recombinant nucleic acid vector construct is introduced into a suitable host cell, and a fifth step, wherein genetically altered host cell clones that produce said monoclonal antibodies are selected and/or identified, using an epitope according to any of preferred embodiments 1 to 6.
18. A monoclonal antibody that is obtained with a method according to preferred embodiment 16 or 17.
19. A method according to preferred embodiment 16 or 17 for the production of a recombinant protein, comprising at least one additional step, wherein the sequence of the nucleic acid encoding said monoclonal antibody is altered in a predetermined way by molecular biological methods.
20. A method according to preferred embodiment 19, wherein the sequence of the nucleic acid encoding said monoclonal antibody is altered by any predetermined point mutations, and/or deletions and/or insertions of nucleic acid fragments of predetermined sequences.
21. A recombinant protein produced with a method according to preferred embodiment 19 or 20, comprising at least one complementarity-determining region of a monoclonal antibody that binds to an epitope according to any of preferred embodiments 1 to 6.
22. A method for the selection and/or identification of a cellular clone producing a monoclonal antibody or a recombinant protein comprising at least one complementarity-determining region of a monoclonal antibody that binds to an epitope according to any of preferred embodiments 1 to 6, wherein an epitope according to any of preferred embodiments 1 to 6 is used.
23. A method for the generation of syngeneic effector T cells for an individual, comprising a first step of isolating T cells from said individual and at least one second step of ex vivo stimulation, wherein an epitope according to any of preferred embodiments 1 to 6 and/or a tumor vaccine according to preferred embodiment 7 or 8 is used.
24. A method according to preferred embodiment 23, wherein T cells are stimulated in the presence of growth factors, in particular IL-2, and/or cytokines that modulate the immune response, in particular IL-4 or IFN-γ.
25. A therapeutical composition comprising a monoclonal antibody according to preferred embodiment 15, and/or preferred embodiment 18 and/or a recombinant protein according to preferred embodiment 21 or obtained in a method according to preferred embodiment 22, and/or syngeneic effector T cells for an individual according to preferred embodiment 24 or 25, and/or a pharmaceutical carrier.
26. A diagnostic composition for use in for the detection of tumor-associated MUC1 antigen within the body of a patient, comprising a monoclonal antibody according to preferred embodiment 15, and/or according to preferred embodiment 18 and/or a recombinant protein according to preferred embodiment 21 or obtained in a method according to preferred embodiment 22.
27. A diagnostic composition according to preferred embodiment 26, for use in a method for the detection of tumor-associated MUC1 antigen comprising an epitope according to any of preferred embodiments 1 to 6.
28. A diagnostic composition according to preferred embodiment 27, for the detection of tumor-associated MUC1 antigen comprising an epitope according to any of preferred embodiments 1 to 6 that is located on tumor cells.
29. A diagnostic composition according to preferred embodiment 27 or 28, for the detection tumor-associated MUC1 antigen comprising an epitope according to any of preferred embodiments 1 to 6 that occurs in free form in the serum of a patient.
30. A diagnostic composition according to any of preferred embodiments 26 to 30, comprising monoclonal antibodies and/or recombinant proteins that carry a detectable label for scintigraphic or nuclear magnetic resonance measurements.
31. A diagnostic composition for use in for the detection of tumor-associated MUC1 antigen in blood and/or tissue samples obtained from the body of a patient, comprising a monoclonal antibody according to preferred embodiment 15, and/or preferred embodiment 18 and/or a recombinant protein according to preferred embodiment 21 or obtained in a method according to preferred embodiment 22.
32. A diagnostic composition according to preferred embodiment 31, for use with tissue samples of any histological origin in the human or animal body.
33. A diagnostic composition according to preferred embodiment 31 or 32, for the detection of epitopes of tumor-associated MUC1 antigen according to any of preferred embodiments 1 to 6.
34. A diagnostic composition according to preferred embodiment 33, for the detection of epitopes located on tumor cells.
35. A diagnostic composition according to preferred embodiment 33 or 34, for the detection of tumor-associated antigen that occurs in free form in the serum of an individual.
36. A diagnostic method, wherein a diagnostic composition according to any of preferred embodiments 31 to 35 is used, comprising at least a first step, wherein tissue samples are brought into contact in vitro with a monoclonal antibody according to preferred embodiment 15, and/or preferred embodiment 18 and/or a recombinant protein according to preferred embodiment 21 or obtained in a method according to preferred embodiment 22, and a second step, wherein specific binding of said monoclonal antibodies and/or proteins to tumor-associated MUC1 antigen in said tissue samples is detected.
37. A diagnostic method according to preferred embodiment 36, wherein a monoclonal antibody according to preferred embodiment 15, and/or preferred embodiment 18 and/or a recombinant protein according to preferred embodiment 21 or obtained in a method according to preferred embodiment 22, carries a detectable label, chosen from the group containing dyes, fluorescent dyes, enzymes catalysing reactions leading to the production of dyes or the emission of electromagnetic energy, a biotin moiety, or epitopes specifically bound by monoclonal antibodies.
38. A method according to preferred embodiment 36 or 37, wherein said monoclonal and/or said recombinant proteins, that have not specifically bound to said tissue samples are removed in washing steps.
39. A BSA-glycopeptide conjugate antigen comprising at least one glycopeptide epitope according to any of preferred embodiments 1 to 6, which comprises an additional cysteine residue at its C-terminal end that is linked by a bifunctional linker molecule, in particular a bi-functional maleimide linker molecule, to bovine serum albumine.
40. Use of a BSA-glycopeptide conjugate antigen according to preferred embodiment 39 for the detection of antibodies and/or cells of the cellular or humoral immune system of an individual, that bind to an epitope according to any of preferred embodiments 1 to 6.
41. A diagnostic method according to preferred embodiment 40, wherein the BSA-glycopeptide conjugate antigen is used to detect of antibodies and/or cells of the cellular or humoral immune system of an individual that bind to an epitope according to any of preferred embodiments 1 to 6, in a blood or tissue sample obtained from an individual.
42. A diagnostic method according to preferred embodiment 41, wherein the BSA-glycopeptide conjugate antigen is used in an ELISA or BiaCore assay.
43. An ELISA or BiaCore assay according to preferred embodiment 42, comprising at least a first step wherein the BSA-glycopeptide conjugate antigen is immobilised on a surface, a second step, wherein a blood or tissue sample obtained from an individual is brought into contact with said surface comprising said immobilised BSA-glycopeptide conjugate antigen, and a third step, wherein antibodies and/or cells of the cellular or humoral immune system in said blood or tissue sample that have bound to said BSA-glycopeptide conjugate antigen are detected.

## Claims

1. Epitopes of tumor-associated MUC1 antigen comprising at least an amino acid sequence according to Sequence No. 1 and comprising at least the carbohydrate moiety at position 3 of Sequence No. 1.

2. Epitopes according to claim 1, wherein the carbohydrate moiety at position 3 is a single αN-Acetyl-Galactosamine residue coupled in O-glycosidic linkage to the threonine or serine residue at position 3.

3. Epitopes according to claims 1 or 2 comprising at least the amino acids and at least the carbohydrate moiety at position 3 of Sequence No. 2.

4. Epitopes according to any of claims 1 to 3, which are specifically bound by monoclonal antibodies 7F11 and/or 1E4, which have been disclosed in the published International Patent Application WO 99/40881.

5. Epitopes according to claim 4 comprising at least the amino acids and at least the carbohydrate moiety at position 3 of Sequence No. 3, and/or comprising at least the amino acids and at least the carbohydrate moiety at position 4 of Sequence No. 4, and/or comprising at least the amino acids and at least the carbohydrate moiety at position 5 of Sequence No. 5, and/or comprising at least the amino acids and at least the carbohydrate moiety at position 3 of Sequence No. 6.

6. Epitopes according to any of claims 1 to 5 comprising at least one additional amino acid of a sequence comprising at least two copies of Sequence No. 6, which are arranged as tandem repeats.

7. A tumor vaccine comprising an epitope according to any of claims 1 to 6, which carries an additional cysteine residue at its C-terminal end, that is linked by a bi-functional linker molecule to a carrier protein.

8. A tumor vaccine according to claim 7, comprising at least one epitope according to Sequence No. 4 or 5, bi-functional maleimide linker molecules and Key-hole Limpet Hemocyanine (KLH).

9. A method for the production of monoclonal antibodies or recombinant proteins, comprising at least a first step, wherein an experimental animal is immunised with an epitope according to any of claims 1 to 6 and/or a tumor vaccine according to claim 7 or 8, and a second step, wherein hybridomas are selected using an epitope according to any of claims 1 to 6 and/or a tumor vaccine according to claim 7 or 8, in particular a modified tumor vaccine comprising bovine serum albumine as carrier protein.

10. A method for the production of a monoclonal antibody or a recombinant protein that binds to an epitope according to any of claims 1 to 6, comprising at least one step, wherein a nucleic acid encoding said monoclonal antibody or recombinant protein is expressed in a vector-host system for the expression of genetic information.

11. A method according to claim 10, comprising at least a first step, wherein a cellular clone that produces said monoclonal antibody or recombinant protein is identified using an epitope according to any of claims 1 to 6, a second step, wherein a nucleic acid encoding said monoclonal antibody or recombinant protein is isolated from said cellular clone, a third step, wherein said nucleic acid or a nucleic acid molecule derived thereof, is ligated to a nucleic acid vector molecule of a vector-host system for the expression of genetic information, a fourth step, wherein the recombinant nucleic acid vector construct is introduced into a suitable host cell, and a fifth step, wherein genetically altered host cell clones that produce said monoclonal antibody or recombinant protein are selected and/or identified, using an epitope according to any of claims 1 to 6.

12. A method according to claim 10 or 11, comprising at least one step, wherein the sequence of the nucleic acid encoding said monoclonal antibody or recombinant protein is altered in a predetermined way by molecular biological methods, comprising in particular the introduction of any predetermined point mutations, and/or deletions and/or insertions of nucleic acid fragments of predetermined sequences.

13. A monoclonal antibody or a recombinant protein comprising at least one complementarity-determining region of a monoclonal antibody that binds to an epitope according to any of claims 1 to 6, that is produced by a genetically altered cell, in particular in transgenic mice expressing the human lg repertoire, in phage display libraries for human antibodies and/or antibody fragments, in human combinatorial antibody libraries producing fully synthetic antibodies, e.g. HuCAL technology, and in recombinant phage antibody systems, e.g. RPAS technology.

14. A method for the selection and/or identification of a genetically altered cell according to claim 13, comprising at least a first step wherein said cell or a sample of a culture comprising said cell or a tissue or ascites sample from a transgenic animal comprising said cell is brought into contact with an epitope according to any of claims 1 to 6, and a second step, wherein binding of a monoclonal antibody or recombinant protein produced by said cell to said epitope is detected.

15. A method according to claims 14, wherein the glycopeptide epitopes according to any claims 1 to 6 comprise bi-functional maleimide linker molecules at their C-terminal ends that are linked to BSA, and are used in ELISA or BiaCore assaies.

16. A method according to claim 15 for the determination of antibodies against MUC1-producing tumours in blood or tissue samples from individuals.

17. Use of a monoclonal antibody and/or a recombinant protein according to any of claims 10-14 in a diagnostic method for the detection of tumor-associated MUC1 antigen within the body of a patient or in tissue samples taken from the body of a patient.

18. A method for the generation of syngeneic effector T cells for an individual, comprising a first step of isolating T cells from said individual and at least one second step of ex vivo stimulation, wherein an epitope according to any of claims 1 to 6 and/or a tumor vaccine according to claim 7 or 8, and/or growth factors, in particular IL-2, and/or immune-modulating cytokines, in particular IL-4 or IFN-γ is used.
